(19) Europäisches Patentamt
European Patent Office
Office européen des brevets

(11) EP 4 467 545 A1

(12) EUROPEAN PATENT APPLICATION
published in accordance with Art. 153(4) EPC

(43) Date of publication:
**27.11.2024 Bulletin 2024/48**

(21) Application number: **22921763.3**

(22) Date of filing: **30.12.2022**

(51) International Patent Classification (IPC):
***C07D 487/04*** *(2006.01)* ***C07D 498/08*** *(2006.01)*
***A61K 31/519*** *(2006.01)* ***A61K 31/5386*** *(2006.01)*
***A61P 35/00*** *(2006.01)*

(52) Cooperative Patent Classification (CPC):
**A61K 31/519; A61K 31/5386; A61P 35/00; C07D 487/04; C07D 498/08**

(86) International application number:
**PCT/CN2022/143770**

(87) International publication number:
**WO 2023/138343 (27.07.2023 Gazette 2023/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.01.2022 CN 202210053350**

(71) Applicant: **Jiangsu Yayo Biotechnology Co. Ltd**
**Nanjing, Jiangsu 210032 (CN)**

(72) Inventors:
- **XIAO, Fei**
  **Nanjing, Jiangsu 210032 (CN)**
- **WENG, Yali**
  **Nanjing, Jiangsu 210032 (CN)**
- **WU, Meng**
  **Nanjing, Jiangsu 210032 (CN)**

(74) Representative: **Høiberg P/S**
**Adelgade 12**
**1304 Copenhagen K (DK)**

(54) **NEW TYPE PYRAZOLOPYRIMIDINE COMPOUND AND COMPOSITION THEREOF, PREPARATION METHOD THEREFOR AND USE THEREOF**

(57) The present invention relates to a new type pyrazolopyrimidine compound, a composition thereof, a preparation method therefor and the use thereof as an anti-cancer drug having anti-tumor activity. The new type pyrazolopyrimidine compound has structural general formula (I) as shown below, is used as an ATR inhibitor, has a good tumor inhibition activity, high selectivity, good water solubility and low toxicity, and can be used for oral administration or intravenous injection administration.

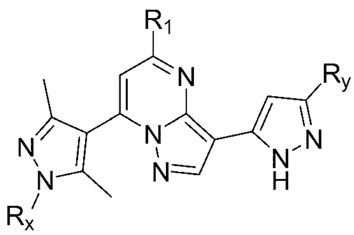

(I)

## Description

## Field of the Invention

The invention pertains to the field of medicines, and relates to novel pyrazolopyrimidine compounds and compositions thereof, preparation methods therefor and use thereof in the preparation of anti-cancer drugs.

## Background of the Invention

Cancer threatens human health and life. In recent years, the research on anti-cancer drugs has shifted towards the development of specific molecular targeted therapeutic drugs.

ATR stands for ataxia telangiectasia and Rad3-related kinase, which is composed of 2644 amino acids. It is an important kinase that can activate cellular responses, block the cell cycle progression, stabilize replication forks and repair DNA after DNA damage, thereby avoiding cell apoptosis. When DNA replication stress and DNA damage occur in the cells, ATRs are recruited to the site of DNA damage, and multiple proteins are involved in regulating the activation of ATRs. Once ATRs are activated, they can regulate cellular biological processes through a variety of signals, including cell cycle arrest, inhibition of replication origins, promotion of deoxynucleotide synthesis, initiation of replication forks, and repair of DNA double-strand breaks.

Due to defects in multiple DNA repair pathways in tumor cells, tumor cells are more dependent on the ATR repair pathway and more sensitive to ATR inhibitors than normal cells. "Synthetic lethality" refers to the defects caused by mutations in one pathway in tumor cells, which make tumor cells more dependent on another complementary pathway than normal cells. Therefore, inhibiting the complementary pathway will cause "synthetic lethality" to tumor cells. Normal cells will not die under drug inhibition because they still have a normal pathway. Studies have found that ATR is a synthetic lethal target for some mutations. For example, tumor cells lacking ATM are more sensitive to ATR inhibitors, and the loss of X-ray repair cross-complementing gene I also makes tumor cells more sensitive to ATR inhibition. Therefore, ATR inhibitors are expected to become excellent potential drugs for tumor treatment due to their characteristics of selectively affecting tumor cells while having less interference with normal cells.

As the most promising "synthetic lethality" therapy after PARP inhibitors, ATR inhibitors have attracted a number of multinational giants to invest in the industry, including Merck, Bayer, AstraZeneca, etc. Among them, Merck's Berzosertib has made the most clinical progress and is currently in Phase II clinical trials. There are a relatively few domestic companies that have developed ATR inhibitors. IMP9064 from Impact Pharmaceutics received FDA approval for Phase I/II clinical studies on October 29, 2021. ATR inhibitors approved for clinical trials by NMPA include Berzosertib from Merck, BAY1895344 from Bayer, and an ATR inhibitor from Shijiazhuang Zhikang Hongren New Drug, all of which are in Phase I clinical trials. A drug currently under clinical development is AZD6738 from AstraZeneca, having a structure as shown below:

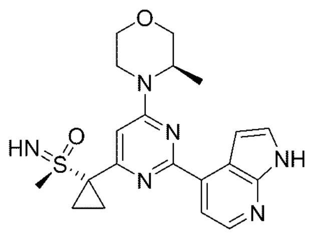
,

which is used as a positive control for the ATR kinase activity array in the experimental part of the present invention. However, to date, no ATR inhibitors have been approved for marketing. Therefore, there is still a need to develop new ATR inhibitors with stronger selectivity and better activity.

## Summary of the Invention

An object of the present invention is to provide a novel ATR inhibitor which has excellent tumor inhibitory activity, strong selectivity, good water solubility, low toxicity, and can be used for oral or intravenous injection administration.

In an aspect, the present invention provides a compound represented by general formula (I):

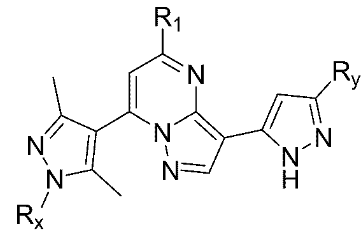

(I)

wherein:

$R_1$ is selected from

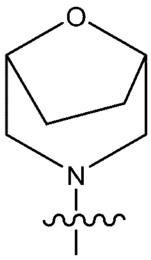,
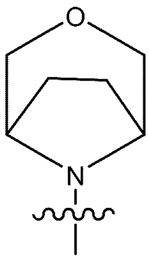,
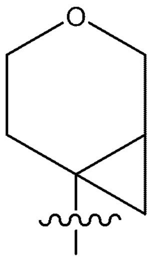,
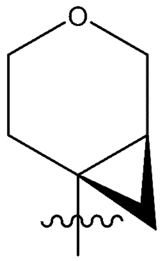,
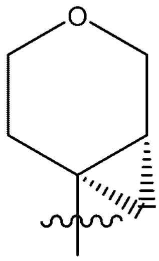,
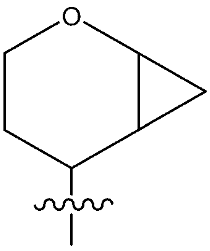,
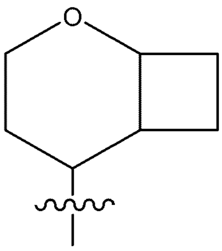,
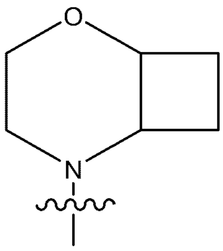,
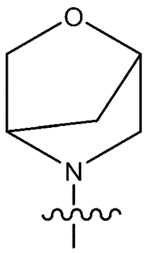,
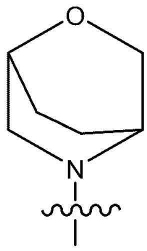,
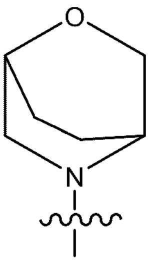,
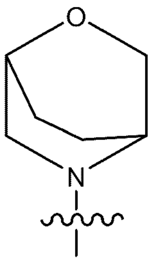,
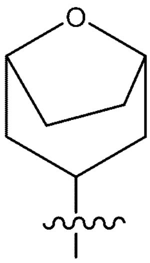,
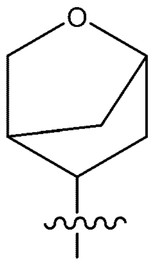,
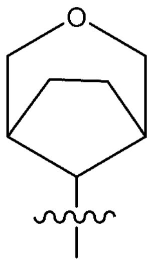, or
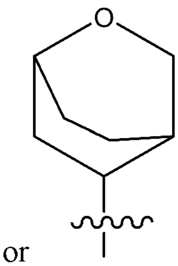;

$R_x$ is selected from H, a 5- to 6- membered heteroaryl, or a 5- to 6- membered heterocyclyl, wherein the 5- to 6- membered heteroaryl or the 5- to 6- membered heterocyclyl is optionally substituted with $R_a$;
$R_a$ is selected from H, halogen, -CN, $-NH_2$, $C_{1-4}$ alkyl, $C_{1-4}$ alkoxy, or $C_{1-4}$ haloalkyl; and
$R_y$ is selected from H, halogen, $NH_2$, $C_{1-4}$ alkyl, or $C_{1-4}$ alkoxy, or
a stereoisomer or a pharmaceutically acceptable salt thereof.

In an embodiment, in the general formula (I), $R_1$ is selected from:

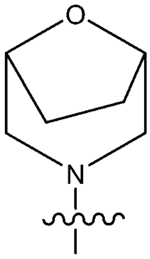, 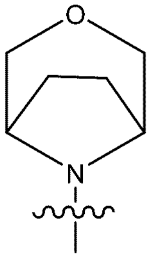, 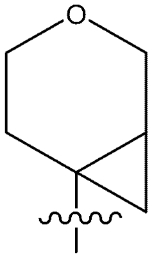, 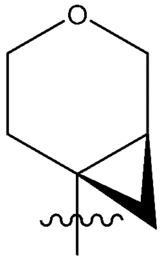, 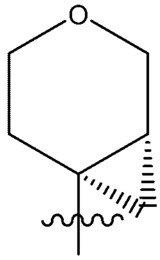, 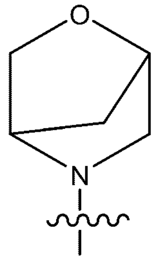, 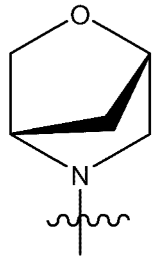,

or

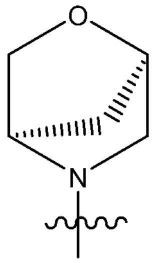

In an embodiment, in the general formula (I), $R_x$ is selected from H, a 6-membered heteroaryl, or a 6-membered heterocyclyl, wherein the 6-membered heteroaryl or the 6-membered heterocyclyl is optionally substituted with $R_a$; and $R_a$ is selected from H, halogen, -CN, $-NH_2$, $C_{1-4}$ alkyl, $C_{1-4}$ alkoxy, or $C_{1-4}$ haloalkyl.

In an embodiment, in the general formula (I), $R_y$ is selected from H or $C_{1-4}$ alkyl.

In an embodiment, the compound has a structure of formula (II):

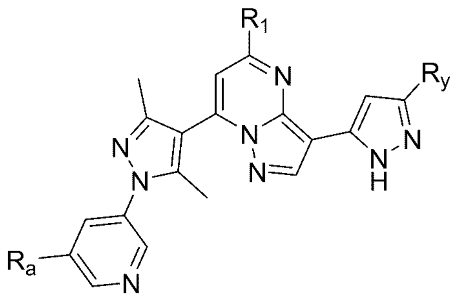

(II)

wherein:

$R_1$ is selected from:

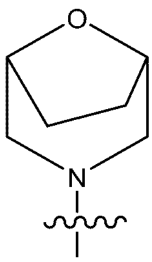, or 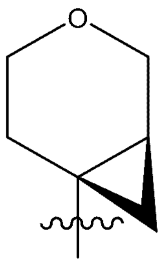;

$R_a$ is selected from H, halogen, -CN, $-NH_2$, $C_{1-4}$ alkyl, $C_{1-4}$ alkoxy, or $C_{1-4}$ haloalkyl; and
$R_y$ is selected from H or methyl.

In an embodiment, the compound of the present invention is selected from:

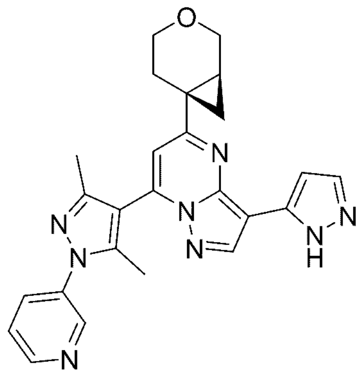 , 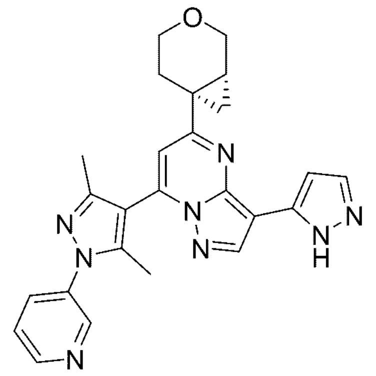 , 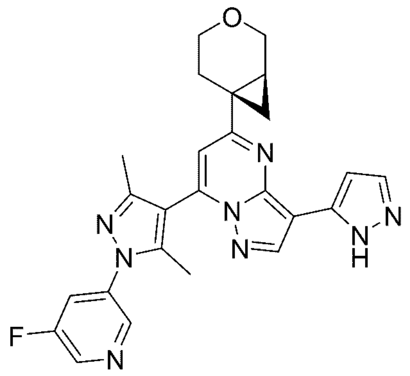 ,

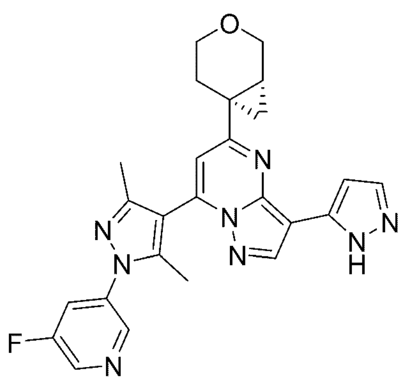 , 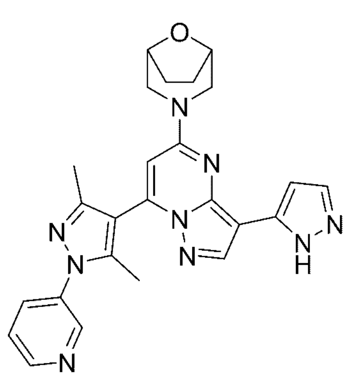 , 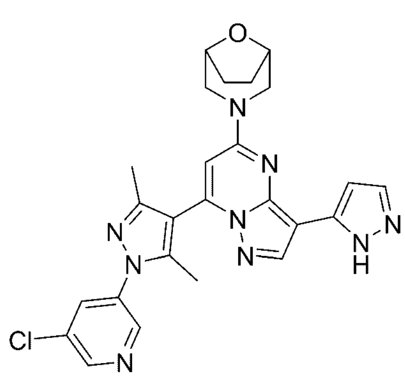 ,

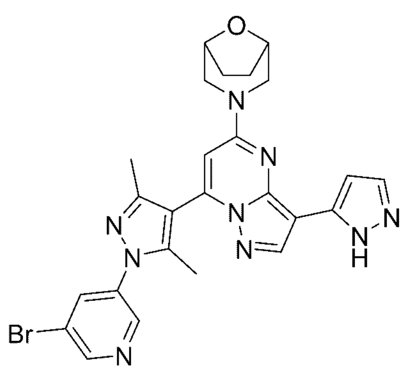 , 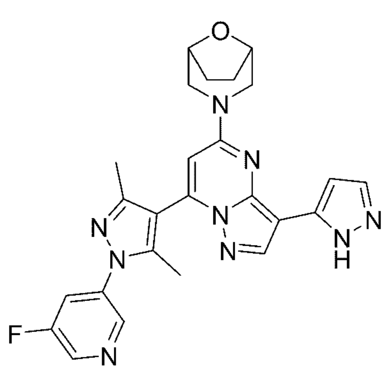 , 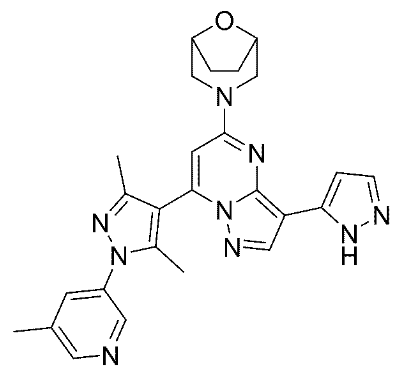 ,

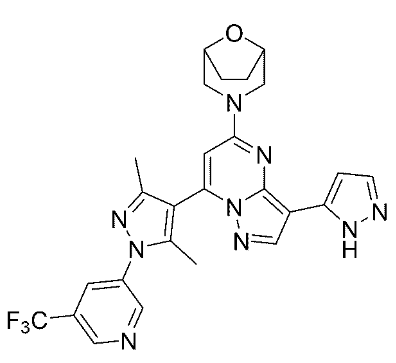 , 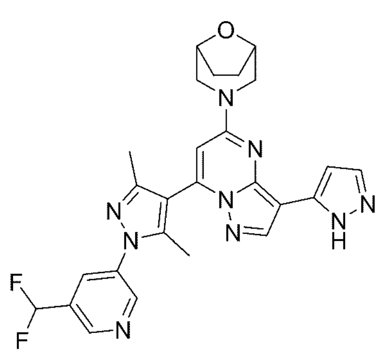 , 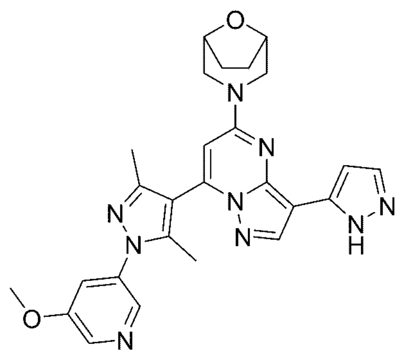 ,

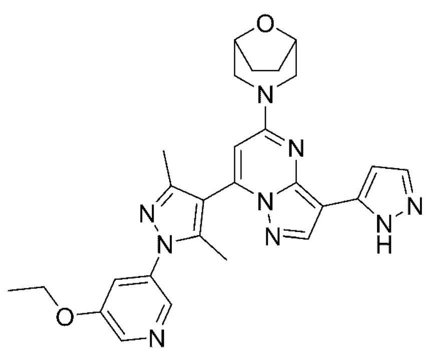
,
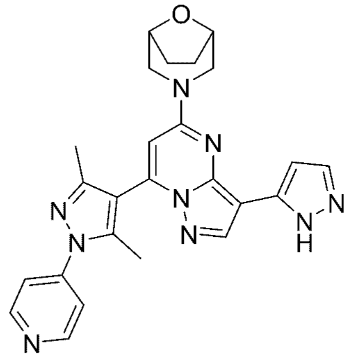
,
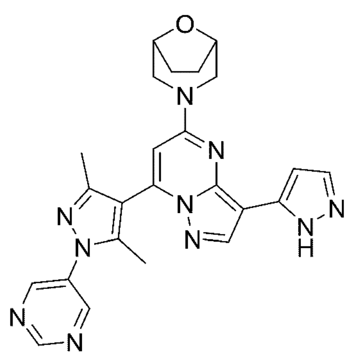
,

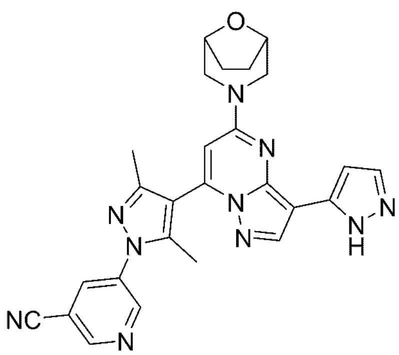
,
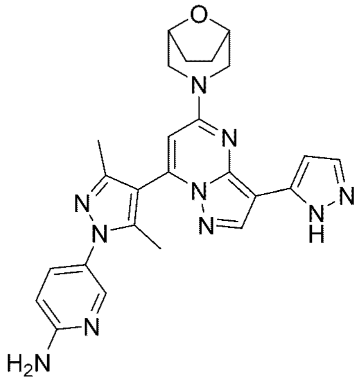
,
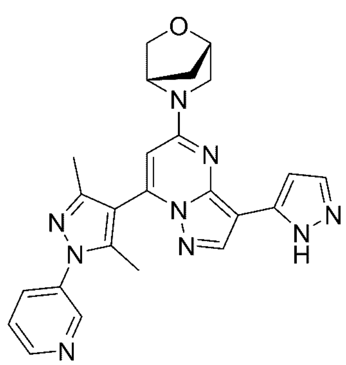
,

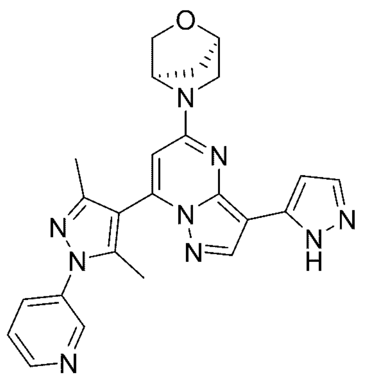
,
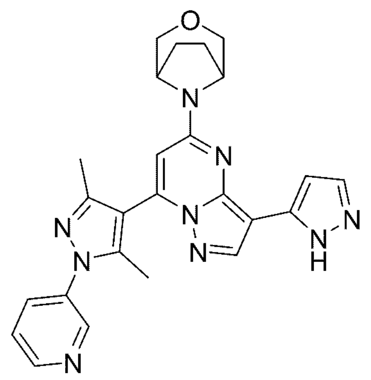
,
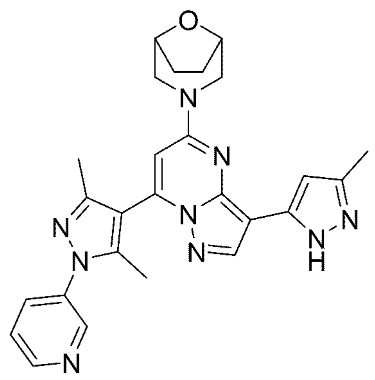
,

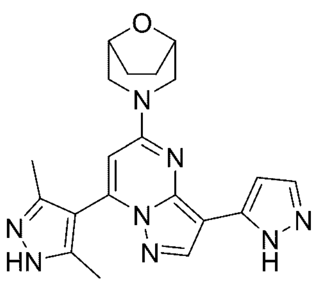
,
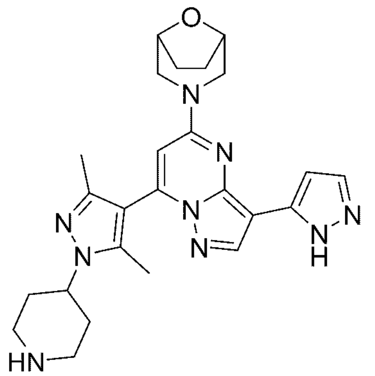
,
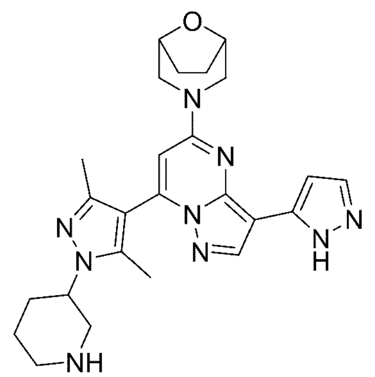
, or

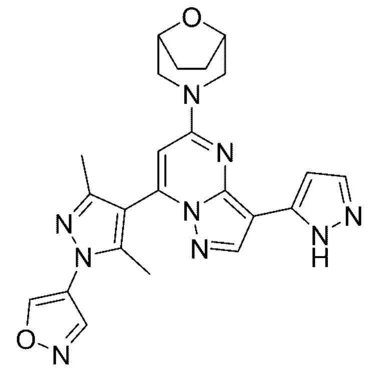

In another aspect, the present invention provides a pharmaceutical composition comprising a therapeutically effective amount of a compound of formula (I) or (II) of the present invention, a stereoisomer or a pharmaceutically acceptable salt thereof, and a pharmaceutically acceptable adjuvant.
In an embodiment, the pharmaceutical composition further comprises an additional active agent useful for treating cancer.
In an embodiment, the pharmaceutical composition can be formulated as an injection, such as sterile aqueous and non-aqueous solutions, a dispersion, a suspension or an emulsion; a solid oral formulation such as tablets, capsules, powders, granules or pills; or a liquid oral formulation such as a solution, an emulsion, a suspension or a syrup.
In another aspect, the present invention provides use of a compound of general formula (I) or (II) of the present invention, a stereoisomer or a pharmaceutically acceptable salt thereof in the preparation of a medicament for treating cancer. The present invention also provides use of the pharmaceutical composition of the present invention in the preparation of a medicament for treating cancer.
In an embodiment, the cancer is selected from a group consisting of breast cancer, kidney cancer, lung cancer, ovarian cancer, bladder cancer, gastric cancer, colorectal cancer, liver cancer, pancreatic cancer, prostate cancer, leukemia, lymphoma, melanoma, myeloma and osteosarcoma.
The technical solutions of the present invention have the following beneficial effects:

1. The present invention provides novel pyrazolopyrimidine compounds.
2. The pyrazolopyrimidine compounds provided by the present invention have an excellent effect of inhibiting the ATR kinase, which is comparable to or even better than that of the clinically investigational drug, AZD6738, indicating that the compounds of the present invention may be used as an ATR inhibitor for treating ATR-mediated diseases.
3. The pyrazolopyrimidine compounds provided by the present invention have an excellent effect of inhibiting the proliferation of a variety of cancer cells and may be useful to treat or prevent a variety of cancers.
4. The pyrazolopyrimidine compounds provided by the present invention have very high stability in mouse and human liver microsomes, indicating that their metabolism *in vivo* is relatively slow.
5. The pyrazolopyrimidine compounds provided by the present invention have a very high *in vivo* exposure after oral administration in mice, and are expected to bring an excellent anti-tumor effect at a lower dose clinically.
6. The pyrazolopyrimidine compounds provided by the present invention is highly selective for ATR and have lower inhibitory activity for other kinases in the family, thereby bringing higher safety benefits.

**Detailed Description of the Invention**

Unless otherwise defined, all technical and scientific terms used herein have the same meaning as commonly understood by those of ordinary skill in the art to which this invention pertains.
It will be understood that both the foregoing summary and the following detailed description are exemplary only for illustration purposes without limiting the subject matter of the present invention in any way.

<u>Definitions</u>

The term "$C_{1-4}$ alkyl" as used herein refers to a straight or branched chain alkyl group having 1 to 4 carbon atoms. Examples thereof include, but are not limited to, methyl, ethyl, propyl, isopropyl, *n*-butyl, isobutyl and *tert*-butyl, with methyl being preferred.
The term "halogen" as used herein refers to fluorine, chlorine, bromine or iodine, preferably fluorine, chlorine or bromine.
The term "$C_{1-4}$ alkoxy" as used herein refers to a group having the formula -O-$C_{1-4}$ alkyl, wherein $C_{1-4}$ alkyl is as

defined above. Examples of $C_{1-4}$ alkoxy include, but are not limited to, methoxy, ethoxy, isopropoxy, *n*-butoxy, isobutoxy, *tert*-butoxy, *etc.*, preferably methoxy and ethoxy.

The term "$C_{1-4}$ haloalkyl" as used herein refers to a $C_{1-4}$ alkyl group substituted with one or more halogen atoms. Examples thereof include, but are not limited to, fluoromethyl, difluoromethyl, trifluoromethyl, fluoroethyl, 1,1-difluoroethyl, chloromethyl, chloroethyl, dichloromethyl, 1,2-dichloroethyl, *etc.*, preferably difluoromethyl and trifluoromethyl.

The term "5- to 6- membered heteroaryl" as used herein refers to a stable 5- to 6-membered aromatic monocyclic group containing 1 to 3, preferably 1 to 2 heteroatoms selected from the group consisting of nitrogen, oxygen and sulfur. The attachment to the heteroaryl group may be at the position of a heteroatom or via a carbon atom of the heterocyclic ring. Examples of the 5-to 6- membered heteroaryl include, but are not limited to, thienyl, furanyl, pyrrolyl, imidazolyl, pyrazolyl, thiazolyl, isothiazolyl, oxazolyl, isoxazolyl, 1,2,3-triazolyl, 1,2,4-triazolyl, 1,2,5-oxadiazole, 1,2,3-oxadiazole, 1,2,4-oxadiazole, 1,3,4-oxadiazole, 1,2,5-thiadiazole, 1,2,3-thiadiazole, 1,2,4-thiadiazole, 1,3,4-thiadiazole, pyridyl, pyrazinyl, triazinyl, pyrimidinyl, pyridazinyl, and the like.

The term "5- to 6- membered heterocyclyl" as used herein refers to a stable 5- to 6-membered non-aromatic monocyclic group containing 1 to 3, preferably 1 to 2 heteroatoms selected from the group consisting of nitrogen, oxygen and sulfur. The heterocyclyl group may be partially or fully saturated. The attachment to the heterocyclyl group may be at the position of a heteroatom or via a carbon atom of the heterocyclic ring. Examples of the 5- to 6- membered heterocyclyl include, but are not limited to, dihydrofuranyl, dihydrothiophenyl, 3-pyrrolinyl, 2-pyrrolinyl, 2-imidazolinyl, 2-pyrazolidinyl, dihydrooxazolyl, dihydrothiazolyl, dihydroisoxazolyl, dihydroisothiazolyl, dihydro-1,2,3-triazolyl, dihydro-1,2,4-triazolyl, dihydro-1,2,5-oxadiazolyl, dihydro-1,2,3-oxadiazolyl, dihydro-1,2,4-oxadiazolyl, dihydro-1,3,4-oxadiazolyl, dihydro-1,2,5-thiadiazolyl, dihydro-1,2,3-thiadiazolyl, dihydro-1,2,4-thiadiazolyl, dihydro-1,3,4-thiadiazolyl, tetrahydrofuranyl, tetrahydrothiophenyl, pyrrolidinyl, imidazolidinyl, pyrazolidinyl, oxazolidinyl, thiazolidinyl, isoxazolidinyl, isothiazolidinyl, 1,2,3-triazolidinyl, 1,2,4-triazolidinyl, 1,2,5-oxadiazolidinyl, 1,2,3-oxadiazolidinyl, 1,3,4-oxadiazolidinyl, 1,2,5-thiadiazolidinyl, 1,2,3-thiadiazolidinyl, 1,3,4-thiadiazolidinyl, 1,2-oxathiolanyl, 1,3-oxathiolanyl, tetrahydropyranyl, tetrahydrothiopyranyl, piperidinyl, 1,4-dioxanyl, morpholinyl, thiomorpholinyl, piperazinyl, and the like.

"Stereoisomer" refer to a compound made up of the same atoms, bonded by the same bonds, but having different three-dimensional structures. The present invention is intended to encompass all kinds of stereoisomers and mixtures thereof.

As used herein, the term "subject" includes mammals and non-mammals. Examples of mammals include, but are not limited to, any member of the class Mammalia: humans, non-human primates (such as chimpanzees and other apes and monkeys); livestock, such as cattle, horses, sheep, goats, and pigs; domestic animals, such as rabbits, dogs and cats; and laboratory animals, including rodents, such as rats, mice and guinea pigs, etc. In some embodiments, the subject is a human.

As used herein, the terms "treat," "treating," "treatment," and other similar synonymous terms include alleviating, reducing or ameliorating the symptoms of, inhibiting (such as preventing the progression of), relieving, improving, alleviating the symptoms caused by, or terminating the symptoms of a disease or condition, preventing additional symptoms, and ameliorating or preventing the underlying metabolic causes that lead to the symptoms. Additionally, the term also encompasses the purpose of prevention. The term also includes the achievement of a therapeutic effect and/or a prophylactic effect. The therapeutic effect refers to the cure or amelioration of the underlying disease being treated. Additionally, cure or amelioration of one or more physiological symptoms associated with the underlying disease is also a therapeutic effect, for example, even if a subject may still be affected by the underlying disease, an improvement in the subject's condition is observed. As for the prophylactic effect, the compounds or compositions of the present invention may be administered to a subject at risk of a particular disease, or to a subject who has not yet been diagnosed with the disease but exhibits one or more physiological symptoms of the disease.

As used herein, the term "therapeutically effective amount" refers to an amount of at least one active substance (e.g., the compound described in the present invention) that is sufficient to alleviate to some extent one or more symptoms of the disease or condition being treated. The result can be reduction and/or alleviation of signs, symptoms, or causes, or any other desired changes in a biological system. For example, a "therapeutically effective amount" is an amount of a composition comprising the compound disclosed herein that is required to provide a clinically significant symptom-alleviating effect. The therapeutically effective amount appropriate in any individual case may be determined using techniques such as dose-escalation trials.

As used herein, the terms "administered," "administration," and the like refer to a method capable of delivering a compound or composition to a desired site for biological action. Such method includes, but is not limited to, an oral route, an intraduodenal route, parenteral injection (including intravenous, subcutaneous, intraperitoneal, intramuscular, intraarterial injection or infusion), topical and rectal administration. Those skilled in the art are familiar with the administration techniques that can be used with the compounds and methods described herein.

As used herein, the term "pharmaceutically acceptable adjuvant" refers to a substance that does not affect the biological activity or properties of the compound of the present invention and is relatively non-toxic, i.e., the substance can be administered to an individual without causing an adverse biological reaction or interacting in an adverse manner with

any of the components contained in the composition. The pharmaceutically acceptable adjuvants include, but are not limited to, carriers, stabilizers, diluents, dispersants, suspending agents, thickeners and/or excipients.

The term "optional" or "optionally" as used herein means that the subsequently described event or circumstance may or may not occur, and that the description includes instances where said event or circumstance occurs and instances where it does not occur. For example, "optionally substituted with $R_a$" means unsubstituted or substituted with $R_a$, and that the description includes both unsubstituted and substituted with $R_a$.

Compounds of general formula (I), stereoisomers or pharmaceutically acceptable salts thereof

The present invention provides compounds for treating, ameliorating or preventing cancer. As described herein, inhibition of ATR kinase can selectively affect tumor cells with less interference with normal cells. The compounds of the present invention can effectively inhibit the activity of ATR kinase, have an effect comparable to or even better than that of the clinically investigational drug AZD6738, and are expected to become excellent potential drugs for tumor treatment.

In an aspect, the present invention provides a compound of formula (I):

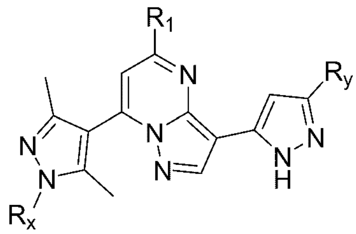

(I)

wherein:

$R_1$ is selected from

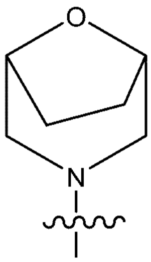 , 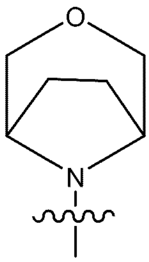 , 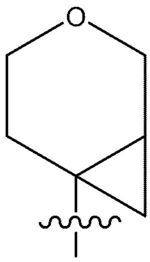 , 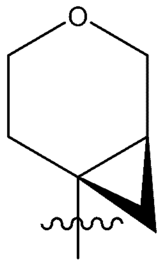 , 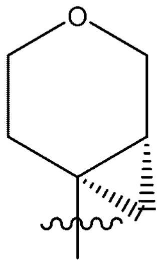 ,

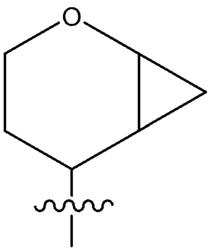 , 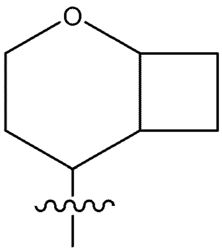 , 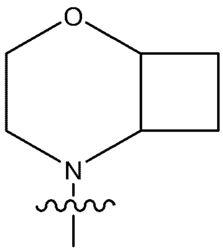 , 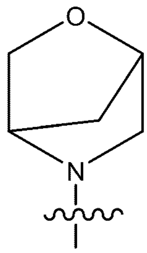 , 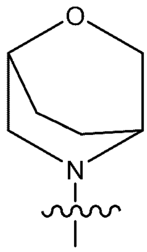 ,

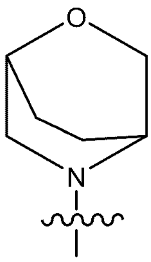 , 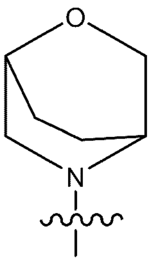 , 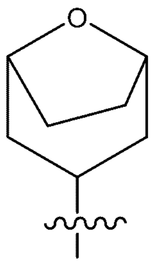 , 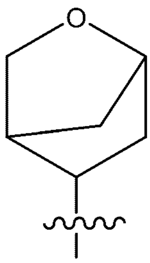 , 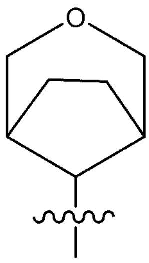 , or 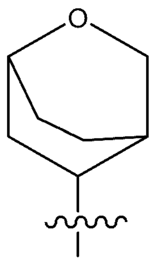 ;

$R_x$ is selected from H, a 5- to 6- membered heteroaryl, or a 5- to 6- membered heterocyclyl, wherein the 5- to 6- membered heteroaryl or the 5- to 6- membered heterocyclyl is optionally substituted with $R_a$;
$R_a$ is selected from H, halogen, -CN, $-NH_2$, $C_{1-4}$ alkyl, $C_{1-4}$ alkoxy, or $C_{1-4}$ haloalkyl; and
$R_y$ is selected from H, halogen, $NH_2$, $C_{1-4}$ alkyl, or $C_{1-4}$ alkoxy, or
a stereoisomer or a pharmaceutically acceptable salt thereof.

In an embodiment, the $R_1$ is selected from:

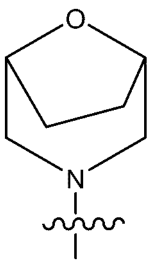 , 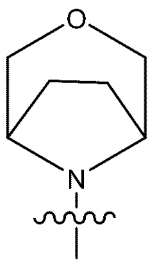 , 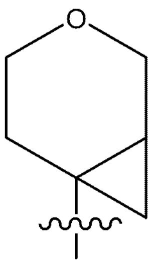 , 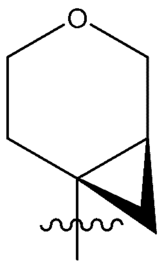 , 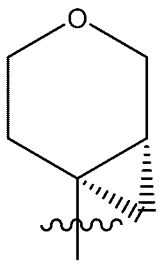 , 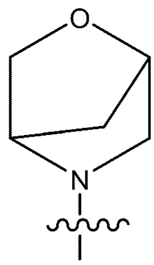 , 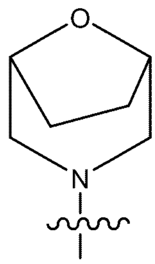 ,

or

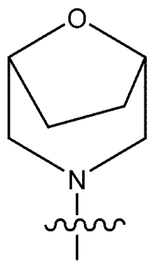

In an embodiment, the $R_1$ is selected from the following groups or stereoisomeric forms thereof (where applicable):

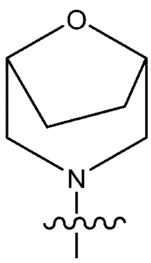 , 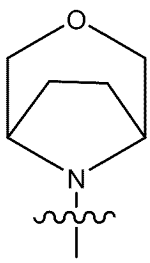 , 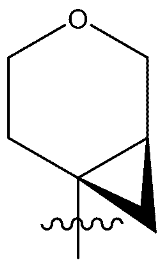 , 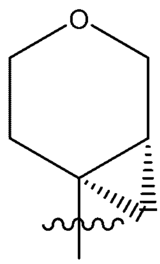 , 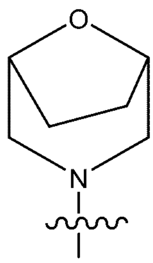 , or 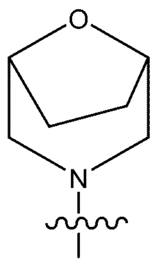

In an embodiment, the $R_1$ is selected from the following groups or stereoisomeric forms thereof (where applicable):

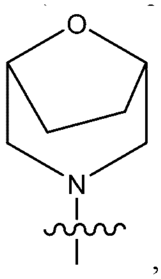 , 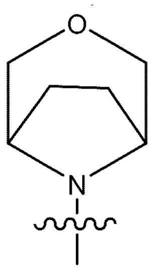 , 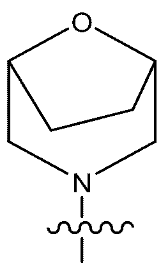 , or 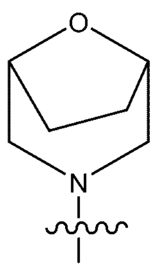

In an embodiment, $R_1$ is selected from

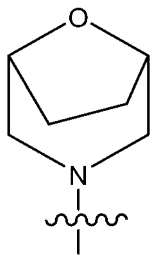 or 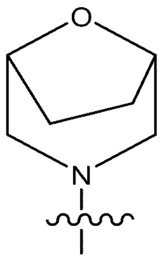

In an embodiment, $R_1$ is

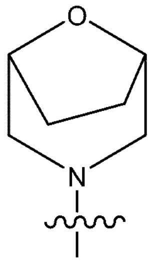

In an embodiment, $R_x$ is selected from H, a 5- to 6- membered heteroaryl, or a 5- to 6-membered heterocyclyl, wherein the 5- to 6- membered heteroaryl or the 5- to 6- membered heterocyclyl is optionally substituted with $R_a$.

In some embodiment, $R_x$ is H.

In some embodiment, $R_x$ is a 5- to 6- membered heteroaryl. In some embodiment, $R_x$ is a 5-to 6- membered heteroaryl group containing 1 or 2 heteroatoms selected from the group consisting of nitrogen, oxygen and sulfur. In some embodiment, $R_x$ is a 5- to 6- membered heteroaryl group containing 1 or 2 heteroatoms selected from the group consisting of nitrogen and oxygen. In some embodiment, $R_x$ is a 6-membered heteroaryl group containing 1 or 2 nitrogen atoms. In some embodiment, $R_x$ is selected from pyridinyl, pyrimidinyl or isoxazolyl. In these embodiments, the heteroaryl group is optionally substituted with $R_a$.

In some embodiments, $R_x$ is a 5- to 6- membered heterocyclyl. In some embodiments, $R_x$ is a 5- to 6- membered heterocyclyl group containing 1 or 2 heteroatoms selected from the group consisting of nitrogen, oxygen and sulfur. In some embodiments, $R_x$ is a 6-membered heterocyclyl group containing 1 or 2 heteroatoms selected from the group consisting of nitrogen and oxygen. In some embodiments, $R_x$ is a 6-membered heterocyclyl group containing 1 or 2 nitrogen atoms. In some embodiments, $R_x$ is a 6-membered heterocyclyl group containing 1 nitrogen atom. In some embodiments, $R_x$ is piperidinyl. In these embodiments, the heterocyclyl is optionally substituted with $R_a$.

In some embodiments, $R_x$ is selected from H,

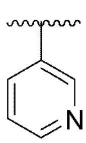, 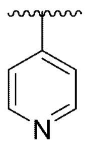, 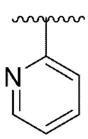, 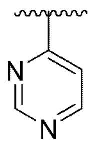, 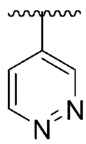,

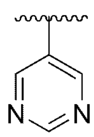, 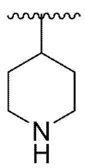, or 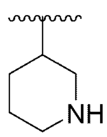,

optionally substituted with $R_a$, wherein  represents the position of attachment to the pyrazole ring.

In some embodiments, $R_x$ is

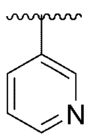,

which is optionally substituted with $R_a$, wherein



represents the position of attachment to the pyrazole ring.

In the aforementioned embodiments, $R_a$ is selected from H, halogen, -CN, -$NH_2$, $C_{1-4}$ alkyl, $C_{1-4}$ alkoxy, or $C_{1-4}$ haloalkyl.

In the aforementioned embodiments, $R_a$ is selected from H, F, Cl, Br, -CN, -$NH_2$, methyl, methoxy, ethoxy, difluoromethyl or difluoroethyl.

In some embodiments, $R_y$ is selected from H, halogen, $NH_2$, $C_{1-4}$ alkyl, or $C_{1-4}$ alkoxy.

In some embodiments, $R_y$ is selected from H or $C_{1-4}$ alkyl.

In some embodiments, $R_y$ is selected from H or methyl.

In an embodiment, in the general formula (I):

$R_1$ is selected from:

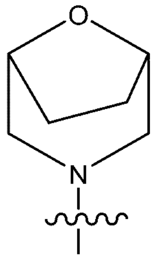 , 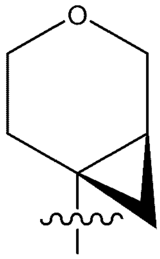 , 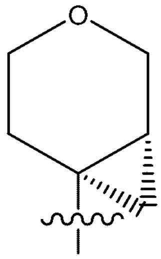 , 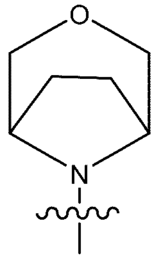 , 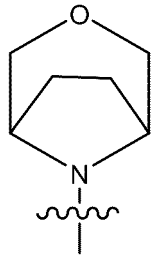 , or 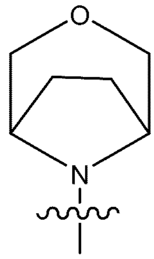 ;

$R_x$ is selected from H, a 5- to 6- membered heteroaryl, or a 6-membered heterocyclyl, wherein the 5- to 6- membered heteroaryl is optionally substituted with $R_a$;

$R_a$ is selected from H, halogen, -CN, -$NH_2$, $C_{1-4}$ alkyl, $C_{1-4}$ alkoxy, or $C_{1-4}$ haloalkyl; and

$R_y$ is selected from H or $C_{1-4}$ alkyl, such as methyl.

In an embodiment, the compound of the present invention has a structure of general formula (II):

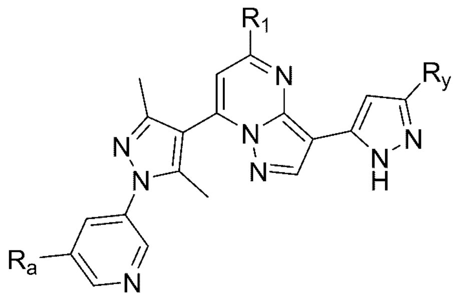

(II)

wherein:

$R_1$ is selected from:

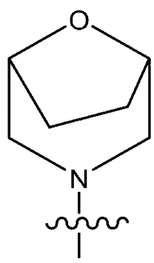 , or 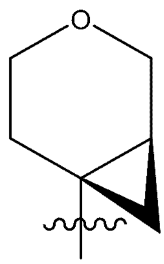 ;

$R_a$ is selected from H, halogen, -CN, $-NH_2$, $C_{1-4}$ alkyl, $C_{1-4}$ alkoxy, or $C_{1-4}$ haloalkyl; and
$R_y$ is selected from H or methyl.

In an embodiment, the compound of general formula (I) of the present invention is selected from:

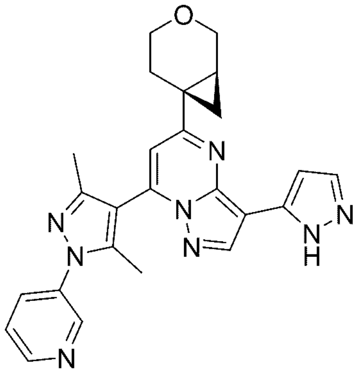
,
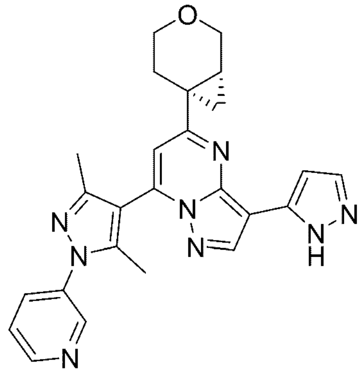
,
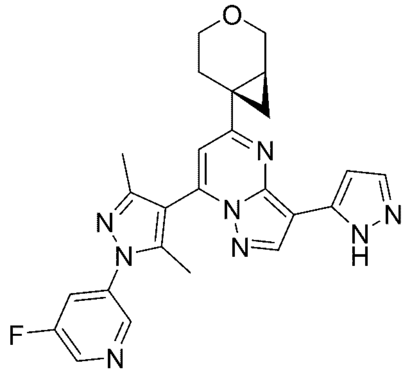
,
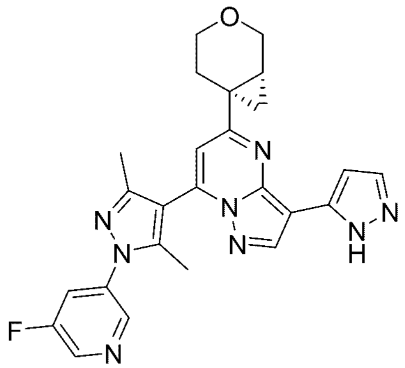
,
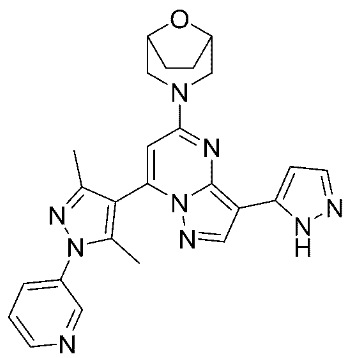
,
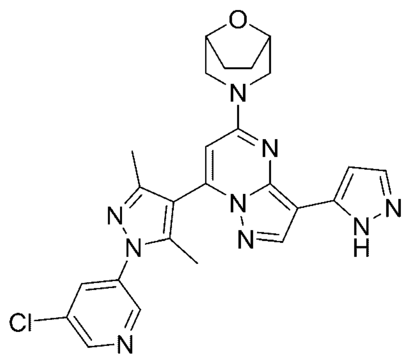
,
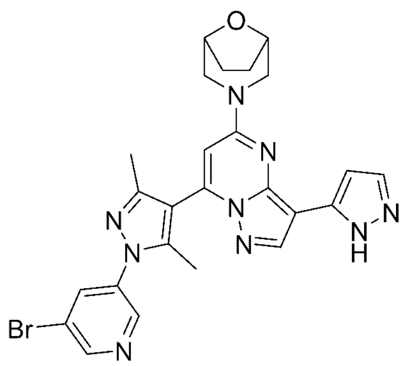
,
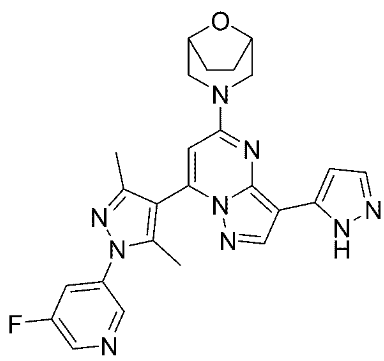
,
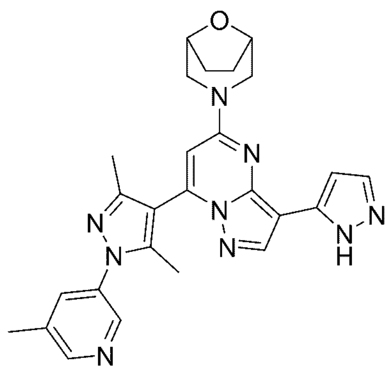
,
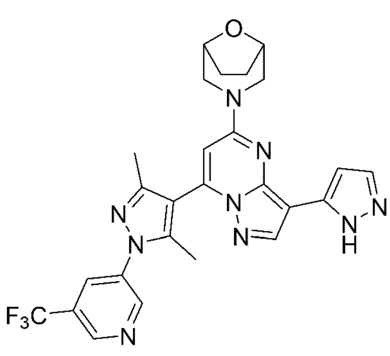
,
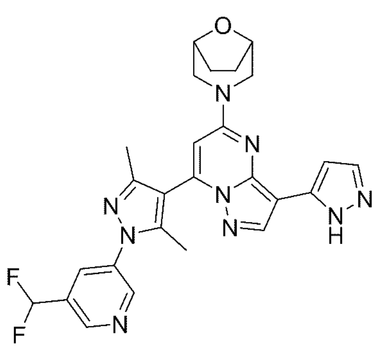
,
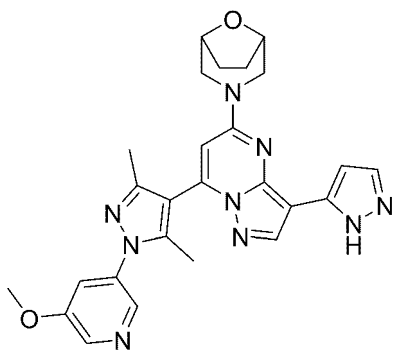
,

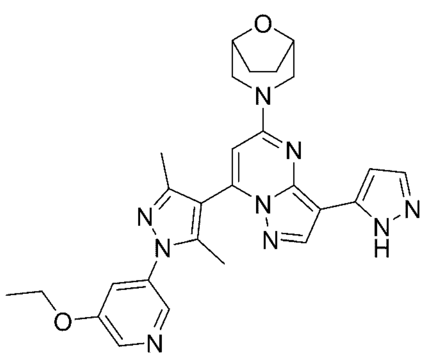 , 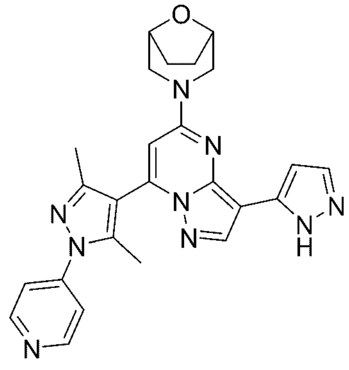 , 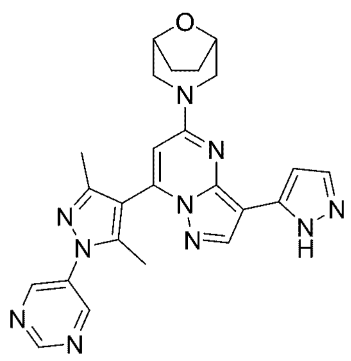 ,

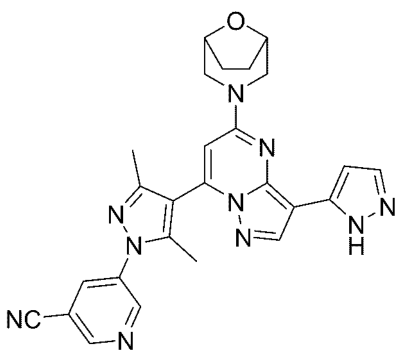 , 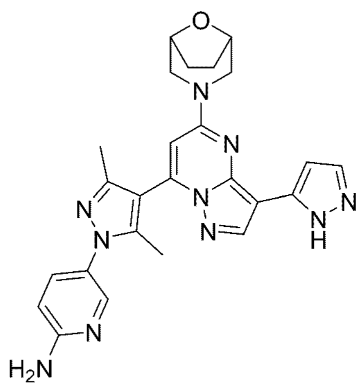 , 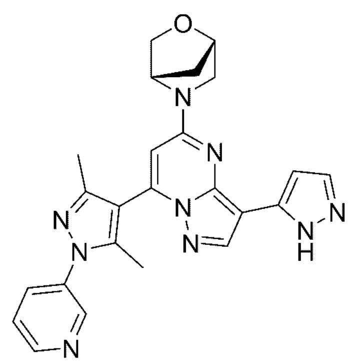 ,

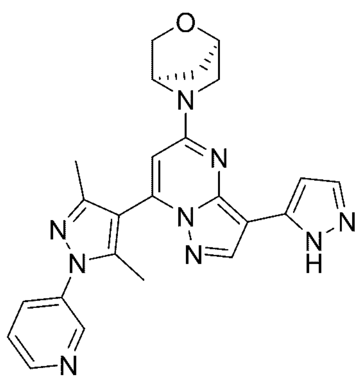 , 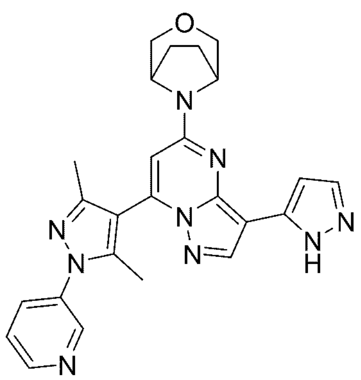 , 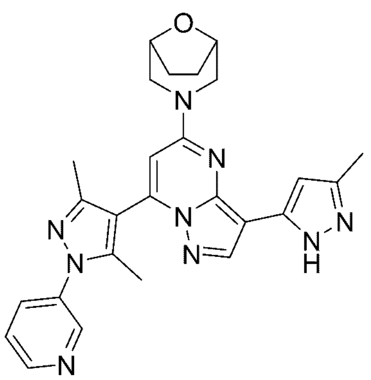 ,

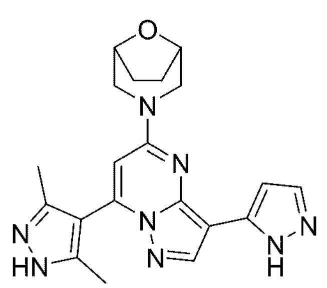 , 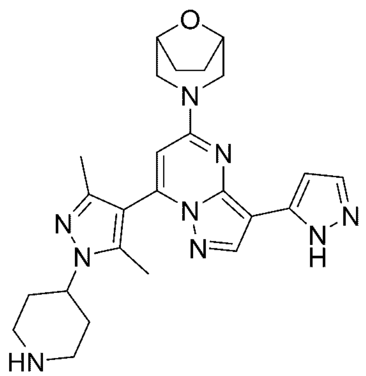 , 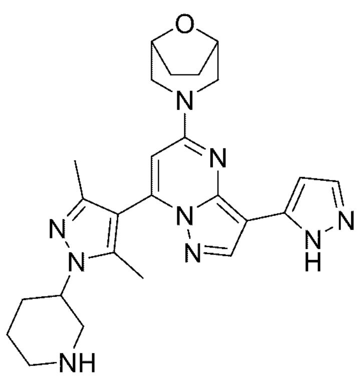 ,

or

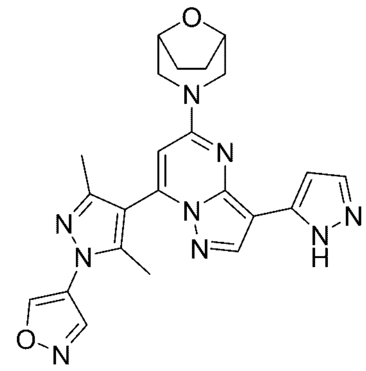

When the compounds of general formula (I) or (II) described in the present invention contain one or more chiral centers, reference to any of these compounds will encompass the enantiomerically pure or diastereomerically pure compounds as well as mixtures of the enantiomers or diastereomers in any ratio, unless otherwise stated.

The compounds of general formula (I) or (II) described in the present invention are usually used in the form of free substances or in the form of pharmaceutically acceptable salts thereof. Pharmaceutically acceptable salts refer to non-toxic, i.e., physiologically acceptable salts. In an embodiment, the pharmaceutically acceptable salts of the compounds of general formula (I) or (II) or stereoisomers thereof described in the present invention include, but are not limited to, hydrochloride, hydrobromide, phosphate, glycerophosphate, nitrite, sulfate, bisulfate, hemisulfate, benzoate, citrate, gluconate, lactate, maleate, succinate, tartrate, acetate, propionate, hexanoate, heptanoate, gluceptate, oxalate, maleate, fumarate, malate, glutamate, pyroglutamate, salicylate, sulfonate (*e.g.*, methanesulfonate, ethanesulfonate, toluenesulfonate and benzenesulfonate), and the like.

Pharmaceutical compositions and pharmaceutical formulations

In an aspect, the present invention further provides a pharmaceutical composition comprising a therapeutically effective amount of a compound of general formula (I) or (II) as defined above, a stereoisomer or a pharmaceutically acceptable salt thereof, and a pharmaceutically acceptable adjuvant.

In the present invention, the term "pharmaceutically acceptable adjuvant" refers to a substance that does not affect the biological activity or properties of the compound of the present invention and is relatively non-toxic, i.e., the substance that can be administered to an individual without causing an adverse biological reaction or interacting in an adverse manner with any components contained in the composition. The pharmaceutically acceptable adjuvants include, but are not limited to, carriers, stabilizers, diluents, dispersants, suspending agents, thickeners and/or excipients.

In an embodiment, the pharmaceutical composition of the present invention may further comprise an additional active agent for treating cancer. Examples of the active agent include, but are not limited to, cisplatin, carboplatin, cyclophosphamide, gemcitabine, Olaparib, topotecan, irinotecan, doxorubicin, paclitaxel, docetaxel, adriamycin, PD-1 or PD-L1 monoclonal antibody drugs (such as nivolumab, atezolizumab, *etc.*).

In an embodiment, the pharmaceutical composition of the present invention may be formulated into a preparation for administration by any of suitable routes. The suitable routes include, but are not limited to, oral route, intraduodenal route, injection (including intravenous, subcutaneous, intraperitoneal, intramuscular, intraarterial injection or infusion), topical and rectal administration, etc.

The pharmaceutical compositions for oral administration include solid oral dosage forms such as tablets, capsules, powders and granules; and liquid oral dosage forms such as solutions, emulsions, suspensions and syrups, as well as powders and granules to be dissolved or suspended in an appropriate liquid.

Solid oral dosage forms may be presented as discrete units (*e.g.*, tablets or hard or soft capsules), each containing a predetermined amount of the active ingredient, and preferably one or more suitable pharmaceutically acceptable adjuvants. Where appropriate, such solid dosage forms can be formulated with coatings, such as enteric coatings, or they can be formulated so as to provide modified release, such as delayed or extended release, of the active ingredient according to methods well known in the art.

Examples of the pharmaceutically acceptable adjuvants suitable for solid oral dosage forms include, but are not limited to, microcrystalline cellulose, corn starch, lactose, mannitol, povidone, croscarmellose sodium, sucrose, cyclodextrin, talc, gelatin, pectin, magnesium stearate, stearic acid, and lower alkyl ethers of cellulose. Similarly, solid formulations may include excipients known in the art for delayed or extended release formulations, such as glyceryl monostearate or hypromellose.

The solid oral dosage forms may be prepared, for example, by mixing the active ingredient with solid excipients and subsequently compressing the mixture in a conventional tablet press; or placing the formulation, for example, in the

form of powders, pills or mini-tablets, into a hard gelatin capsule.

The liquid oral dosage forms may be presented, for example, as solutions, emulsions, suspensions, elixirs, syrups, oral drops or liquid-filled capsules. The liquid oral dosage forms may also be presented as powders for dissolution in an aqueous or non-aqueous liquid to form a solution or suspension prior to use. Examples of the excipients suitable for liquid oral formulations include, but are not limited to, ethanol, propylene glycol, glycerol, polyethylene glycol, poloxamers, sorbitol, polysorbates, mono- and diglycerides, cyclodextrins, coconut oil, palm oil, and water. The liquid oral dosage forms may be prepared, for example, by dissolving or suspending the active ingredient in an aqueous or non-aqueous liquid or by incorporating the active ingredient into an oil-in-water or water-in-oil liquid emulsion.

The pharmaceutical compositions for parenteral administration may also be in the form of a sterile injectable formulation, such as sterile aqueous and non-aqueous solutions, a dispersion, a suspension or an emulsion for injection or infusion, a concentrate for injection or infusion, and a sterile powder to be reconstituted in a sterile solution or dispersion for injection or infusion prior to use. Examples of the pharmaceutically acceptable adjuvants suitable for parenteral formulations include, but are not limited to, water, coconut oil, palm oil, cyclodextrin solution, Ringer's solution, isotonic sodium chloride solution, and the like. Sterile injectable formulations may be formulated according to known techniques using appropriate pharmaceutically acceptable adjuvants.

The pharmaceutically acceptable adjuvants used in any pharmaceutical formulation must be suitable for the intended route of administration and compatible with the active ingredient.

In an embodiment, the pharmaceutical composition of the present invention may be formulated as an injection, preferably an intravenous injection. In an embodiment, the pharmaceutical composition of the present invention may be formulated as sterile aqueous and non-aqueous solutions, a dispersion, a suspension or an emulsion for injection.

In an embodiment, the pharmaceutical composition of the present invention may be formulated as a solid oral formulation. In an embodiment, the pharmaceutical composition of the present invention may be formulated as tablets. In an embodiment, the pharmaceutical composition of the present invention may be formulated as capsules, including hard capsules or soft capsules. In an embodiment, the pharmaceutical composition of the present invention may be formulated as a powder. In an embodiment, the pharmaceutical composition of the present invention may be formulated as granules. In an embodiment, the pharmaceutical composition of the present invention may be formulated as pills.

In an embodiment, the pharmaceutical composition of the present invention may be formulated as a liquid oral formulation. In an embodiment, the pharmaceutical composition of the present invention may be formulated as an oral solution, an emulsion, a suspension or a syrup.

## Uses

The compounds of general formula (I) or (II) of the present application, stereoisomer or pharmaceutically acceptable salts thereof, or the pharmaceutical compositions of the present invention may effectively inhibit the activity of ATR kinase with an effect comparable to or even better than that of the clinically investigational drug AZD6738, indicating that they may be useful to treat ATR kinase-mediated diseases.

The compounds of general formula (I) or (II) of the present invention, stereoisomers or pharmaceutically acceptable salts thereof, or the pharmaceutical compositions of the present invention may effectively inhibit the proliferation of tumor cells and thus may be useful to treat tumors or cancers.

In an aspect, the present invention provides use of a compound of formula (I) or (II) of the present invention, a stereoisomer or a pharmaceutically acceptable salt thereof, or a pharmaceutical composition of the present invention in the preparation of a medicament for treating cancer in a subject.

In an embodiment, the cancer includes, but is not limited to, breast cancer, kidney cancer, lung cancer, ovarian cancer, bladder cancer, gastric cancer, colorectal cancer, liver cancer, pancreatic cancer, prostate cancer, leukemia, lymphoma, melanoma, myeloma, and osteosarcoma.

In an embodiment, the cancer is breast cancer. In an embodiment, the cancer is renal cancer. In an embodiment, the cancer is lung cancer, such as non-small cell lung cancer. In an embodiment, the cancer is ovarian cancer. In an embodiment, the cancer is bladder cancer. In an embodiment, the cancer is gastric cancer. In an embodiment, the cancer is colorectal cancer. In an embodiment, the cancer is liver cancer. In an embodiment, the cancer is pancreatic cancer. In an embodiment, the cancer is prostate cancer. In an embodiment, the cancer is leukemia. In an embodiment, the cancer is lymphoma, such as mantle cell lymphoma. In an embodiment, the cancer is melanoma. In an embodiment, the cancer is myeloma. In an embodiment, the cancer is osteosarcoma.

## Preparation methods

In an aspect, the present invention provides a method for preparing a compound of general formula (I) of the present invention:

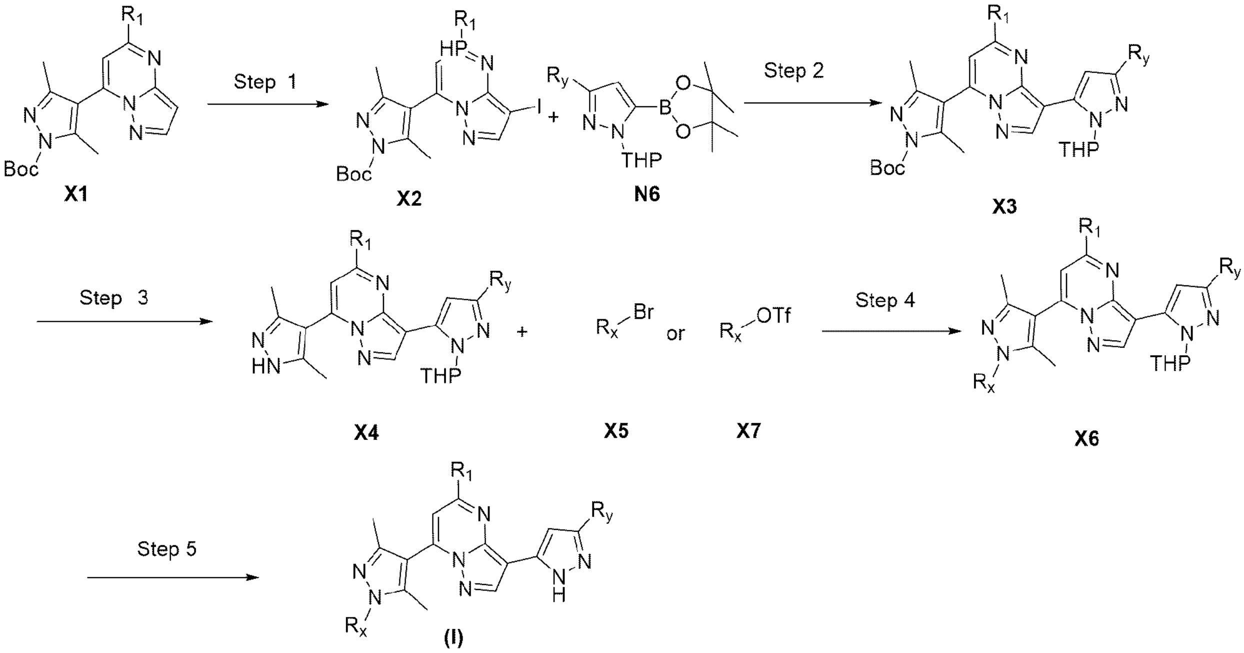


X1 X2 N6 X3

X4 X5 or X7 X6

(I)

Specifically, the method comprises:

Step 1: Reacting compound **X1**

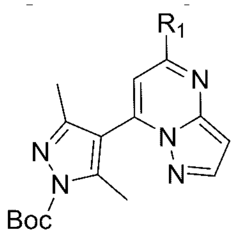

**X1**

with N-iodosuccinimide NIS to obtain compound **X2**

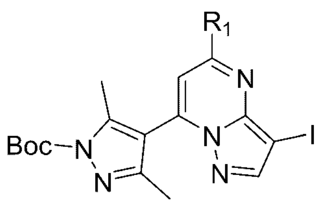

**X2** ;

Step 2: subjecting compound **X2** and compound **N6**

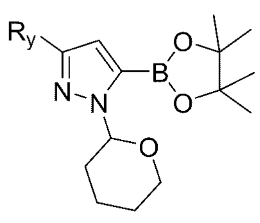

**N6**

to Sukuzi reaction to obtain compound **X3**

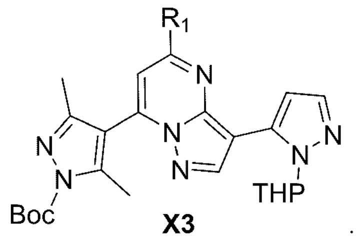

**X3**

;

Step 3: Removing the Boc protecting group from compound **X3** to obtain compound **X4**

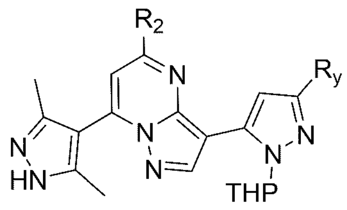

**X4**

;

Step 4: subjecting compound **X4** and compound **X5** (or compound **X7**)

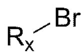

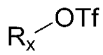

**X5** **X7**

to Ullmann reaction (or substitution reaction to obtain compound **X6**

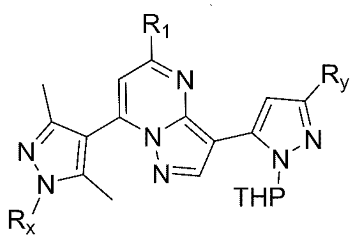

**X6**

;

and

Step 5: removing the THP protecting group from compound **X6** to obtain the compound of general formula (I) of the present invention.

In an embodiment, an exemplary reaction scheme for preparing a compound of general formula (I) of the present invention is provided:

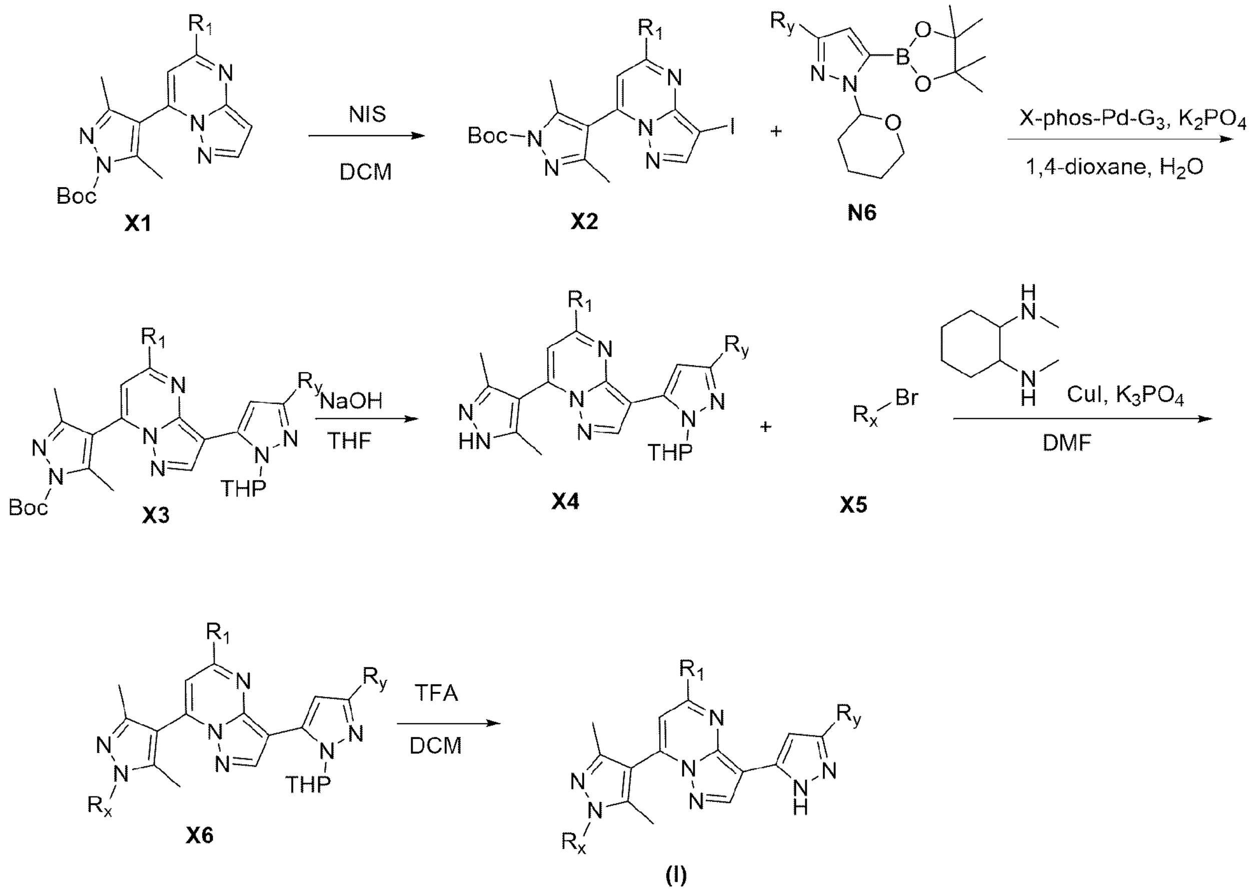


X1 X2 N6 X3 X4 X5 X6 (I)

wherein $R_1$, $R_x$ and $R_y$ are as defined in the general formula (I).

According to the above scheme, compound **X1** is used as a starting material and reacted with N-iodosuccinimide NIS in a suitable solvent such as dichloromethane to obtain compound **X2**; compound **X2** is reacted with 1-(2-tetrahydropyranyl)-1H-pyrazole-5-boronic acid pinacol ester analogue **N6** in a suitable solvent such as a mixture solution of 1,4-dioxane/water in the presence of potassium phosphate and Xphos-Pd-G3 to obtain compound **X3**; compound **X3** is reacted with a base such as NaOH in a suitable solvent such as tetrahydrofuran to obtain compound **X4**; compound **X4** is reacted with compound **X5** in the presence of potassium phosphate, CuI and N,N-dimethyl-1,2-cyclohexanediamine to obtain compound **X6**; and compound **X6** is deprotected under an acidic condition to obtain the compound of general formula (I).

In an embodiment, a method for preparing a compound of general formula (II) of the present invention is provided:

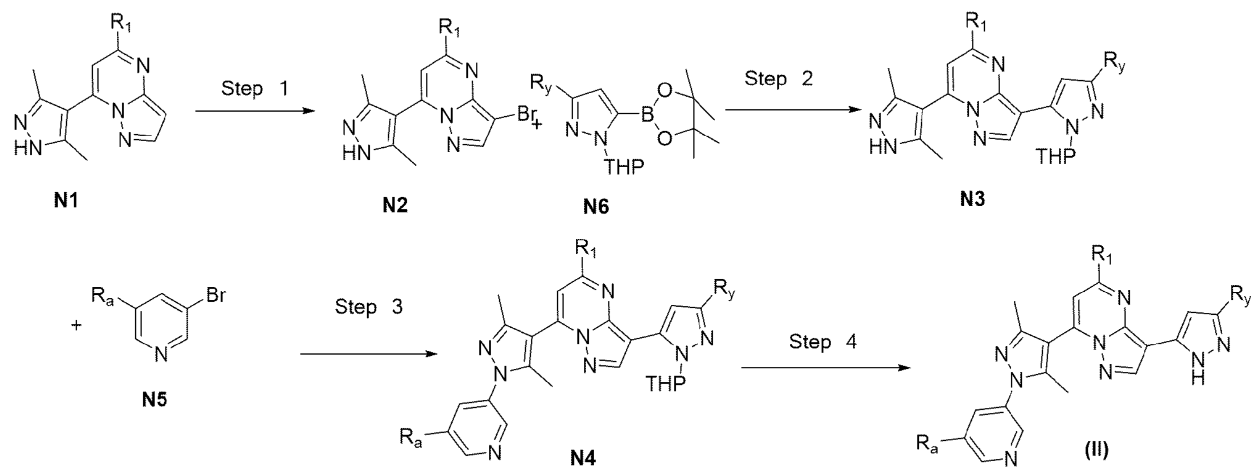


N1 N2 N6 N3 N5 N4 (II)

Step 1: reacting compound **N1**

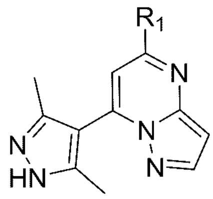

**N1**

with N-bromosuccinimide NBS to obtain compound **N2**

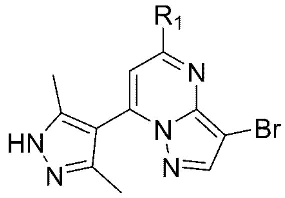

**N2** ;

Step 2: subjecting compound **N2** and compound **N6**

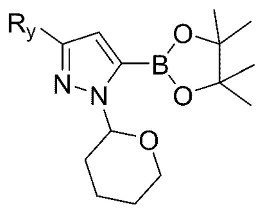

to Suzuki reaction in an appropriate solvent to obtain compound **N3**

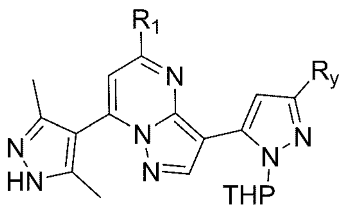

**N3** ;

Step 3: subjecting compound **N3** and compound **N5**

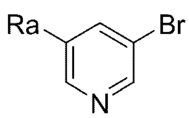

**N5**

to Ullmann reaction to obtain compound **N4**

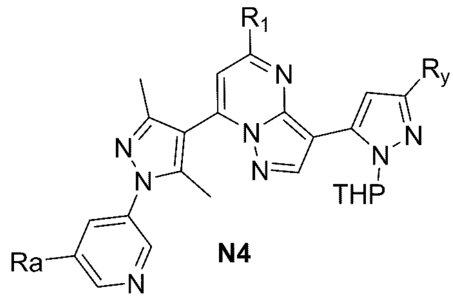

N4

;

and

Step 4: removing the THP protecting group from compound **N4** to obtain the compound of general formula (II) of the present invention.

In an embodiment, an exemplary reaction scheme for preparing a compound of general formula (II) of the present invention is provided:

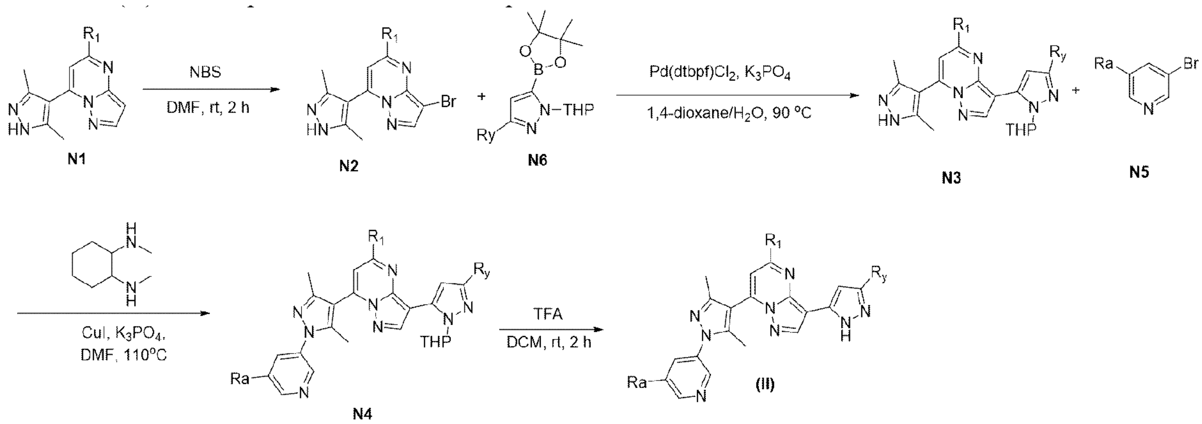


According to the above scheme, compound **N1** is used as a starting material to react with N-bromosuccinimide NBS in a suitable solvent such as DMF to obtain compound **N2**; compound **N2** is reacted with 1H-pyrazole-5-boronic acid pinacol ester analogue **N6** in a suitable solvent such as a mixture solution of 1,4-dioxane/water in the presence of potassium phosphate and Pd(dtbpf)$Cl_2$ to obtain compound **N3**; compound **N3** is reacted with compound **N5** in the presence of potassium phosphate, CuI and N,N-dimethyl-1,2-cyclohexanediamine to obtain compound **N4**; and compound **N4** is deprotected under an acidic condition to obtain the compound of general formula (II).

Stereoisomers of the compounds of general formula (I) or (II) of the present invention may be obtained by separating the obtained racemic mixture or other mixtures based on their different physicochemical properties by well-known means, such as fractional crystallization or HPLC technology. Enantiomers may also be separated using chiral chromatography columns. It is also possible to use an optically active starting material and react it under a condition which does not cause racemization or epimerization.

The pharmaceutically acceptable salt of a compound of general formula (I) or (II) of the present invention or a stereoisomer thereof may be formed by reacting the N atom in the structure of the compound or the stereoisomer thereof with an inorganic or organic acid by methods known in the art. The inorganic acid includes, but is not limited to, hydrochloric acid, hydrobromic acid, phosphoric acid, glycerophosphoric acid, hemisulfuric acid, sulfuric acid, hydrogen sulfate, hydroiodic acid, nitrous acid, and the like. The organic acid includes, but is not limited to, benzoic acid, citric acid, gluconic acid, lactic acid, maleic acid, succinic acid, tartaric acid, acetic acid, propionic acid, hexanoic acid, heptanoic acid, glucoheptanoic acid, oxalic acid, maleic acid, fumaric acid, malic acid, glutamic acid, pyroglutamic acid, salicylic acid, sulfonic acids (*e.g.*, methanesulfonic acid, ethanesulfonic acid, toluenesulfonic acid, and benzenesulfonic acid), and the like.

Other intermediates or reactants that are not mentioned in detail may be purchased or prepared by synthetic methods generally known in the art.

The above general synthetic routes only represent general methods of most examples. For a compound with a special substituent, changes known to those skilled in the art can be made in a certain step of the reaction or the reaction sequence can be changed.

## Examples

The following examples illustrate the preparation and biological activity evaluation of the compounds of the present invention.

The following examples are provided to enable those skilled in the art to more clearly understand and practice the present invention. They should not be considered as limiting the scope of the invention, but merely as being illustrative and representative.

The materials and reagents used herein are either commercially available or prepared by synthetic methods generally known in the art. The following reaction schemes illustrate specific methods for synthesizing the compounds of the present invention.

Details are as follows:

Preparation of key intermediates **a1-a3**:

Preparation of intermediate **a1**:

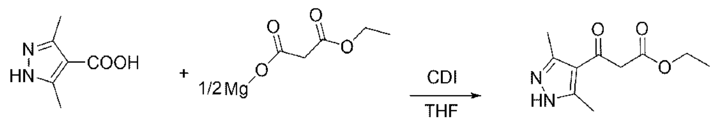


a1-1 a1-2 a1

Intermediate **a1-1** (60.6 mmol, 8.5 g) and carbonyldiimidazole (CDI, 19.6 g, 121.2 mmol) were dissolved in 100 mL of anhydrous tetrahydrofuran. After reacting at room temperature for 2 hours, the mixture was warmed to 55 °C, continued to stir for 4 hours, and then cooled to room temperature. To the reaction solution intermediate **a1-2** (17.2 g, 121.2 mmol) was slowly added in portions and the mixture was warmed to 55 °C to continue the reaction for 40 hours. The reaction was stopped, filtered, evaporated to remove the solvent under reduced pressure, added with 50 mL of water, extracted with ethyl acetate, separated by flash column chromatography to obtain intermediate **a1** (9.1 g, yield: 72%). LC-MS: $[M+H]^+$: 211.

Preparation of intermediate **a2**:

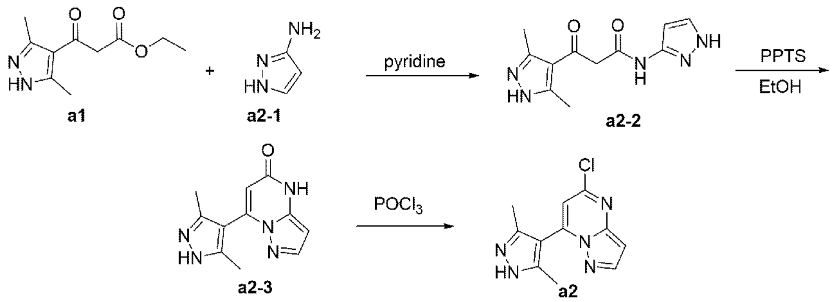


a1 a2-1 a2-2 a2-3 a2

Step 1: Intermediate **a1** (43.3 mmol, 9.1 g) was dissolved in 60 mL of pyridine, added with 3-aminopyrazole **a2-1** (34.0 mmol, 2.82 g), heated to 110°C for 12 hours, and then cooled to room temperature. The mixture was evaporated to remove the solvent under reduced pressure to obtain 9.0 g of crude intermediate **a2-2**. LC-MS: $[M+H]^+$: 248.

Step 2: The crude intermediate **a2-2** (9.0 g) was dissolved in 80 mL of ethanol, added with pyridine p-toluenesulfonate **PPTS** (54.6 mmol, 13.7 g), heated to 90°C, and reacted for 36 hours, and then the reaction was stopped. The solvent was evaporated under reduced pressure. The system was added with 100 mL of water, extracted with ethyl acetate, and separated by flash column chromatography to obtain intermediate **a2-3** (7.5 g, two-step yields: 76%). LC-MS: $[M+H]^+$: 230.

Step 3: Under an ice bath, intermediate **a2-3** (19.6 mmol, 4.5 g) was dissolved in 25 mL of phosphorus oxychloride. After stirring for 10 minutes, the ice bath was removed, the temperature was raised to 110°C and stirring

was continued for 2 hours. The reaction was stopped, and cooled to room temperature. To the reaction solution 150 mL of ice water was slowly added, adjusted to about pH-8 with a saturated sodium bicarbonate solution, extracted with ethyl acetate, and dried over anhydrous sodium sulfate. The crude product was separated by flash column chromatography to obtain intermediate **a2** (3.5 g, yield: 72%). LC-MS: $[M+H]^+$: 248.

Preparation of intermediate **a3**:

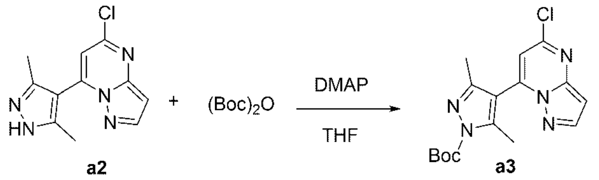

a2 + $(Boc)_2O$ → a3

Intermediate **a2** (6.01 mmol, 1.5 g) and 4-dimethylaminopyridine DMAP (0.6 mmol, 73 mg) were dissolved in 20 mL of tetrahydrofuran, and di-tert-butyl dicarbonate (9.01 mmol, 1.97 g) was slowly added. After reacting at room temperature for 2 hours, the reaction was stopped. The system was added with 50 mL of water, extracted with ethyl acetate, and dried over anhydrous sodium sulfate to obtain intermediate **a3** (1.9 g, yield: 91%), LC-MS: $[M+H]^+$: 348.

Preparation of key intermediate **b1**:

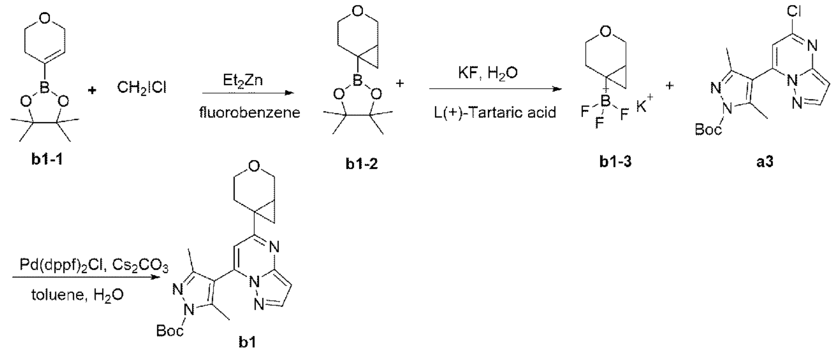

b1-1 + $CH_2ICl$ → b1-2 → b1-3 + a3 → b1

Step 1: Under nitrogen protection, and at -5°C, raw material **b1-1** (5 mmol, 1.05 g) was dissolved in 15 mL of fluorobenzene, slowly added with diethylzinc (2 mmol/L in toluene, 12.5 mL, 25 mmol) and a solution of chloroiodomethane in fluorobenzene (4.4 g/1.8 mL, 25 mmol), and reacted at room temperature for 12 hours. The system was placed in an ice bath, added with 40 mL of a saturated aqueous ammonium chloride solution to quench the reaction, extracted with ethyl acetate, dried over anhydrous sodium sulfate, and separated by flash column chromatography to obtain intermediate **b1-2** (730 mg, yield: 65%).

Step 2: Under nitrogen protection, the intermediate **b1-2** (3.23 mmol, 730 mg) was dissolved in 16 mL of a mixture of methanol/acetonitrile (1/1, v/v), and potassium fluoride aqueous solution (749 mg/3 mL) was slowly added. After the mixture was stirred for 10 minutes, L-tartaric acid (6.46 mmol, 968 mg) and tetrahydrofuran (350 μL) were added and the reaction was continued at room temperature for 1.5 h. The reaction was stopped, filtered, and the filtrate was concentrated to obtain intermediate **b1-3** (600 mg, crude).

Step 3: Under nitrogen protection, intermediate **b1-3** (7.9 mmol, 1.6 g), intermediate **a3** (7.9 mmol, 2.75 g) and cesium carbonate (23.7 mmol, 7.7 g) were dissolved in 30 mL of a mixture solution of toluene/water (6/1, v/v), and Pd(dppf) $Cl_2$ (0.8 mmol, 586 mg) was added. The mixture was heated to 110°C and reacted for 12 hours. The reaction was stopped and filtered. The system was added with 70 mL of water, extracted with ethyl acetate, dried over anhydrous sodium sulfate, and separated by flash column chromatography to obtain intermediate **b1** (1.3 g, yield: 41%). LC-MS: $[M+H]^+$: 410.

Preparation of key intermediates **c1-c8**:

Preparation of intermediate **c1**:

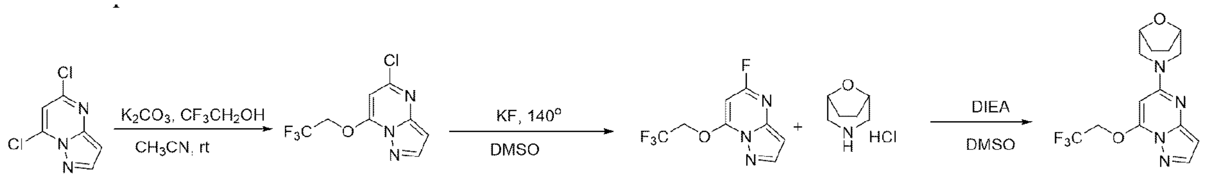


c1-1 c1-2 c1-3 c1

Step 1: Raw material **c1-1** of 5,7-dichloropyrazolo[1,5-a]pyrimidine (37.2 mmol, 7.0 g) and potassium carbonate (55.85 mmol, 7.72 g) were dissolved in 50 mL of acetonitrile, added with trifluoroethanol (40.96 mmol, 4.1 g), and reacted at room temperature for 16 hours. Then the reaction was stopped. The solvent was evaporated under reduced pressure. The mixture was added with 100 mL of water, extracted with ethyl acetate, dried over anhydrous sodium sulfate, and concentrated. The crude product was purified by column chromatography (PE/EA = 6/1) to give compound **c1-2** (7.90 g, yield: 84%). LC-MS: $[M+H]^+$: 252.0.

$^1H$ NMR (400 MHz, DMSO-$d_6$) $\delta$ 8.28 (d, $J$ = 2.0 Hz, 1H), 7.01 (s, 1H), 6.72 - 6.70 (m, 1H), 5.36 (q, $J$ = 8.4 Hz, 2H).

Step 2: Compound **c1-2** of 5-chloro-7-(2,2,2-trifluoroethoxy)pyrazolo[1,5-a]pyrimidine (31.4, 79.0 g) and potassium fluoride (157.0 mmol, 9.12 g) were dissolved in 80 mL of dry DMSO, heated to 140° C for 4 hours, and then cooled to room temperature to stop the reaction. The mixture was added with 100 mL of water, extracted with ethyl acetate, washed with saturated brine, dried over anhydrous sodium sulfate, and concentrated. The crude product was purified by column chromatography (PE/EA = 10/1) to give compound **c1-3** (3.5 g, yield: 47%). LC-MS: $[M+H]^+$: 236.1.

Step 3: Compound **c1-3** of 5-fluoro-7-(2,2,2-trifluoroethoxy)pyrazolo[1,5-a]pyrimidine (14.88 mmol, 3.5 g), N,N-diisopropylethylamine DIEA (44.65 mmol, 5.77 g) and 8-oxa-3-azabicyclo[3.2.1]octane hydrochloride (14.88 mmol, 2.23 g) were dissolved in 80 mL of dry DMSO, stirred for 5 minutes, heated to 120° C and continued to react for 2 hours, and then cooled to room temperature to stop the reaction. The reaction solution was added with 120 mL of water, extracted with ethyl acetate, washed with saturated brine, dried over anhydrous sodium sulfate, and concentrated. The crude product was purified by column chromatography (PE/EA=10/1) to give compound **c1** (3.0 g, yield: 61%). LC-MS: $[M+H]^+$: 329.0.

$^1H$ NMR (400 MHz, DMSO-$d_6$) $\delta$ 7.87 (d, $J$ = 2.0 Hz, 1H), 6.25 (s, 1H), 6.05 (d, $J$ = 2.0 Hz, 1H), 5.21 (q, $J$ = 8.8 Hz, 2H), 4.45 (d, $J$ = 2.4 Hz, 2H), 4.02 (d, $J$ = 12.4 Hz, 2H), 3.08 (dd, $J$ = 12.8 Hz, 2.0 Hz, 2H), 1.87 - 1.81 (m, 2H), 1.74 - 1.67 (m, 2H).

Referring to the synthesis of intermediate **c1**, similar raw materials/intermediates were used to synthesize intermediates **c2 to c4** below:

| **Raw materials/ Intermediates used** | **Structure of intermediate product** | **LC-MS/$^1$H NMR** |
|---|---|---|
| The structure of the raw material used in Step 3: 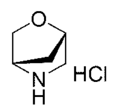 | 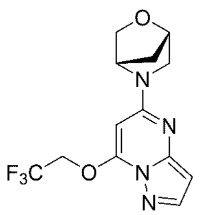 c2 | $[M+H]^+$ = 315.0.<br>$^1H$ NMR (400 MHz, DMSO-$d_6$) $\delta$ 7.85 (d, $J$ = 2.0 Hz, 1H), 6.03 (br s, 2H), 5.20 (q, $J$ = 8.8 Hz, 2H), 4.96 (s, 1H), 4.71 (s, 1H), 3.81 (d, $J$ = 7.2 Hz, 1H), 3.70 (d, $J$ = 7.2 Hz, 1H), 3.51 (d, $J$ = 10.4 Hz, 1H), 3.40 (d, $J$= 9.6 Hz, 1H), 1.92 (q, $J$ = 9.6 Hz, 2H). |

(continued)

| Raw materials/ Intermediates used | Structure of intermediate product | LC-MS/$^1$H NMR |
|---|---|---|
| The structure of the raw material used in Step 3: 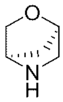 HCl | 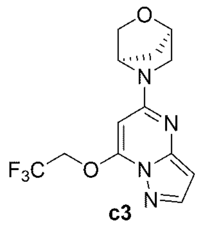 **c3** | $[M+H]^+$ = 315.1. $^1$H NMR (400 MHz, DMSO-$d_6$) $\delta$ 7.85 (d, *J* = 2.0 Hz, 1H), 6.03 (br s, 2H), 5.20 (q, *J* = 8.8 Hz, 2H), 4.96 (s, 1H), 4.71 (s, 1H), 3.81 (d, *J* = 7.2 Hz, 1H), 3.69 (d, J = 7.2 Hz, 1H), 3.51 (d, *J* = 10.4 Hz, 1H), 3.40 (d, *J* = 10.0 Hz, 1H), 1.92 (q, *J* = 10.0 Hz, 2H). |
| The structure of the raw material used in Step 3: 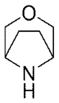 | 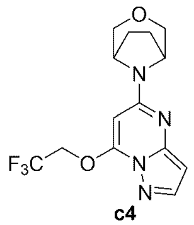 **c4** | $[M+H]^+$ = 329.3. $^1$H NMR (400 MHz, DMSO-$d_6$) $\delta$ 7.88 (d, *J* = 2.4 Hz, 1H), 6.31 (s, 1H), 6.07 (d, *J* = 2.0 Hz, 1H), 5.20 (q, *J* = 8.8 Hz, 2H), 4.59 (s, 2H), 3.67 (d, *J* = 10.8 Hz, 2H), 3.58 (d, *J* = 10.4 Hz, 2H), 2.04 - 1.90 (m, 4H). |

Preparation of intermediate **c5**:

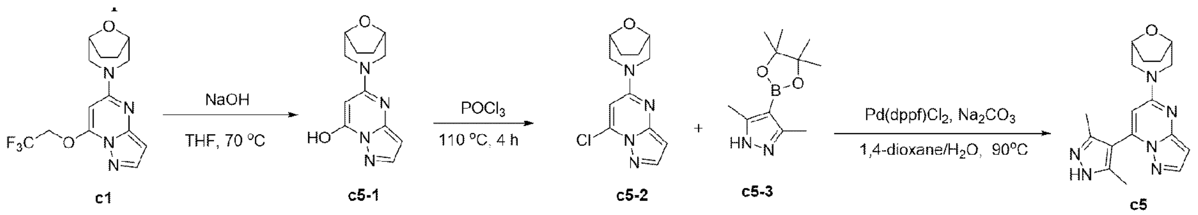

c1 c5-1 c5-2 c5-3 c5

Step 1: Intermediate **c1** of 3-(7-(2,2,2-trifluoroethoxy)pyrazolo[1,5-a]pyrimidin-5-yl)-8-oxa-3-azabicyclo[3.2.1]octane (9.14 mmol, 3.0 g) and sodium hydroxide (18.28 mmol, 0.73 g) were dissolved in 20 mL of tetrahydrofuran, heated to 70° C for 16 hours, and cooled to room temperature. The reaction was stopped, and filtered. The solvent was evaporated under reduced pressure, and the crude product was separated and purified by flash reverse column chromatography (acetonitrile/water) to obtain compound **c5-1** (2.0 g, yield: 89%). LC-MS: $[M+H]^+$: 247.2.

$^1$H NMR (400 MHz, DMSO-$d_6$) $\delta$ 7.48 (d, *J* = 2.0 Hz, 1H), 5.65 (d, *J* = 1.6 Hz, 1H), 4.89 (s, 1H), 4.35 (s, 2H), 3.71 (d, *J* = 12.4 Hz, 2H), 2.83 (dd, J = 12.0 Hz, 1.6 Hz, 2H), 1.81 - 1.68 (m, 4H).

Step 2: Compound **c5-1** of 5-(8-oxa-3-azabicyclo[3.2.1]octan-3-yl)pyrazolo[1,5-a]pyrimidine-7-hydroxy (8.12 mmol, 2.00 g) was dissolved in 10 mL of phosphorus oxychloride, heated to 110°C for 4 hours, and cooled to room temperature. The reaction was stopped. The reaction solution was slowly poured into 50 mL of an ice-water mixture, and a saturated aqueous sodium bicarbonate solution was added to adjust the pH to about 9. The mixture was extracted with ethyl acetate, washed with saturated brine, dried over anhydrous sodium sulfate, and concentrated. The crude product was separated and purified by column chromatography (PE/EA = 6/1) to give compound **c5-2** (1.3 g, yield: 61%). LC-MS: $[M+H]^+$: 265.1.

$^1$H NMR (400 MHz, DMSO-$d_6$) $\delta$ 7.98 (d, *J* = 2.4 Hz, 1H), 7.03 (s, 1H), 6.19 (d, *J* = 2.4 Hz, 1H), 4.44 (d, *J* = 2.0 Hz, 2H), 4.00 (d, *J* = 12.4 Hz, 2H), 3.10 (dd, *J* = 12.4 Hz, 2.0 Hz, 2H), 1.87-1.81 (m, 2H), 1.74 - 1.66 (m, 2H).

Step 3: Under nitrogen protection, compound **c5-2** of 3-(7-chloropyrazolo[1,5-a]pyrimidin-5-yl)-8-oxa-3-azabicyclo[3.2.1]octane (4.91 mmol, 1.30 g), sodium carbonate (14.73 mmol, 1.56 g), raw material **c5-3** (1.64 g, 7.37 mmol) and catalyst Pd(dppf)$Cl_2$ (0.49 mmol, 0.36 g) were dissolved in 10 mL of 1,4-dioxane, added with 2 mL of water, stirred for 5 minutes, heated to 90 °C and continued to react for 16 hours, and cooled to room temperature. The reaction was stopped, and filtered. The mixture was added with 35 mL of water, extracted with ethyl acetate, washed with saturated brine, dried over anhydrous sodium sulfate, and concentrated. The crude product was purified by column chromatography (DCM/MeOH=10/1) to give compound **c5** (0.7 g, yield: 44%). LC-MS: $[M+H]^+$: 325.3.

$^1H$ NMR (400 MHz, DMSO-$d_6$) $\delta$ 12.63 (s, 1H), 7.84 (d, $J$ = 2.4 Hz, 1H), 6.50 (s, 1H), 6.09 (d, $J$ = 2.0 Hz, 1H), 4.43 (s, 2H), 4.01 (d, $J$ = 13.2 Hz, 2H), 3.08 (dd, J = 12.4 Hz, 2.0 Hz, 2H), 2.18 (d, $J$ = 28.0 Hz, 6H), 1.89 - 1.81 (m, 2H), 1.77 - 1.69 (m, 2H).

Referring to the synthesis of intermediate **c5**, similar raw materials/intermediates were used to synthesize intermediates **c6 to c8** below:

| **Raw materials/ Intermediates used** | **Structure of product** | **LC-MS/$^1$H NMR** |
|---|---|---|
| The structure of the intermediate used in Step 1: **c2** | 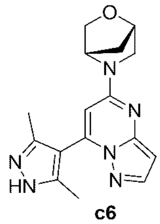 **c6** | $[M+H]^+$ = 311.1. |
| The structure of the intermediate used in Step 1: **c3** | 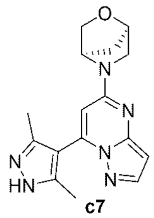 **c7** | $[M+H]^+$ = 311.2. |
| The structure of the intermediate used in Step 1: **c4** | 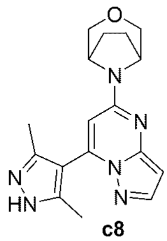 **c8** | $[M+H]^+$ = 325.2. |

**Example 1:** Preparation of Compound **M3**

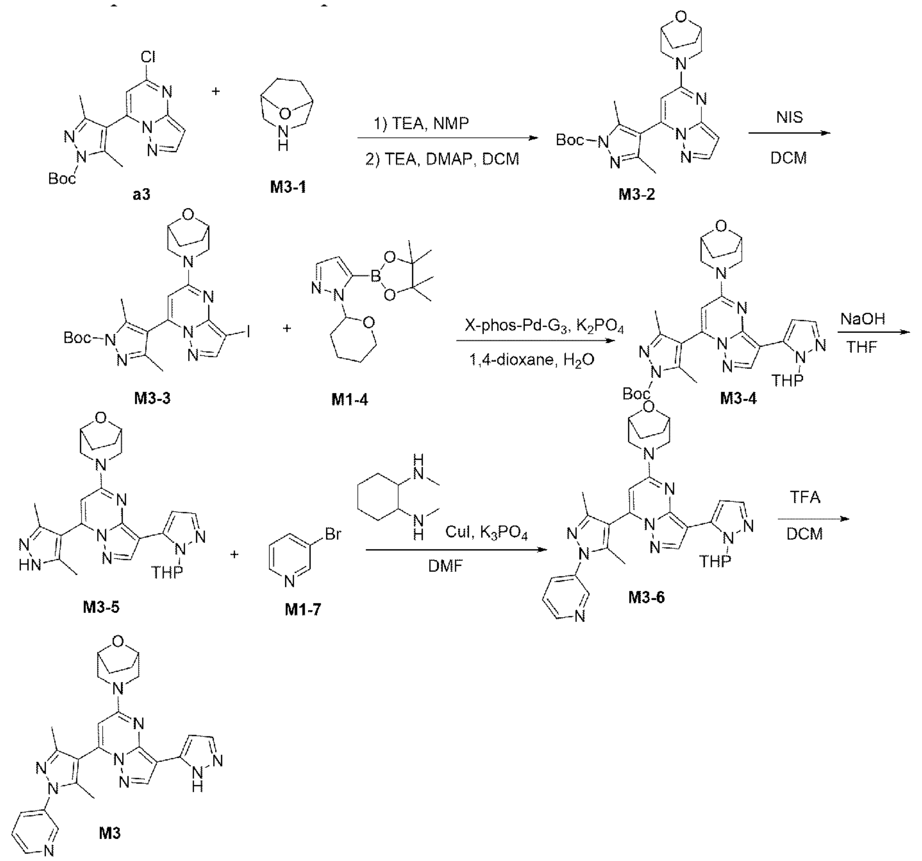


Intermediate **a3** (1.29 mmol, 450 mg) and raw material **M3-1** (1.43 mmol, 161 mg) were dissolved in 10 mL of N-m ethyl pyrrolidone, added with triethylamine (2.60 mmol, 263 mg), and heated in a microwave at 180° C for 2 hours. The reaction was stopped, and to the system 40 mL of water was added, and extracted with dichloromethane. The mixture and 4-dimethylaminopyridine **DMAP** (0.06 mmol, 8 mg) were dissolved in 12 mL of dichloromethane, added with triethylamine (1.29 mmol, 131 mg) and di-*tert*-butyl dicarbonate (1.29 mmol, 282 mg), and reacted at room temperature for 2 hours, and then the reaction was stopped. The system was added with 30 mL of water, extracted with dichloromethane, and dried over anhydrous sodium sulfate to obtain a crude intermediate **M3-2**. LC-MS: $[M+H]^+$: 425.

Under an ice bath, the crude intermediate **M3-2** from the previous step was dissolved in 12 mL of anhydrous dichloromethane, and N-iodosuccinimide NIS (1.29 mmol, 290 mg) was slowly added in portions, and continued to react under stirring for 2 hours. The reaction was stopped. 30 mL of a saturated aqueous ammonium chloride solution was added, and extracted with dichloromethane. After drying over anhydrous sodium sulfate, the product was separated by flash column chromatography (PE/EA = 2/1) to obtain compound **M3-3** (450 mg, two-step yields: 64%). LC-MS: $[M+H]^+$: 551.

Under nitrogen protection, intermediate **M3-3** (0.82 mmol, 450 mg) and raw material **M1-4** (1.23 mmol, 342 mg) were dissolved in 10 mL of a mixture solution of 1,4-dioxane and water (v/v: 9/1), and potassium phosphate (1.64 mmol, 348 mg) and Xphos-Pd-G3 (0.08 mmol, 68 mg) were added. The reaction was heated in a microwave at 95 °C for 2 hours. The reaction was stopped, and filtered. The system was added with 30 mL of water, extracted with ethyl acetate, dried over anhydrous sodium sulfate, and separated by flash column chromatography (PE/EA = 2/1) to obtain compound **M3-4** (260 mg, yield: 56%). LC-MS: $[M+H]^+$: 575.

Intermediate **M3-4** (260 mg) was dissolved in 10 mL of tetrahydrofuran, added with 5 mL of 1N NaOH aqueous solution, heated to 70° C and reacted for 1 hour. The reaction was stopped, and the solvent was evaporated under reduced pressure. 40 mL of water was added, extracted with dichloromethane, and separated by flash column chromatography (PE/EA = 1/1) to obtain compound **M3-5** (200 mg, yield: 94%). LC-MS: $[M+H]^+$: 475.

Under nitrogen protection, intermediate **M3-5** (0.42 mmol, 200 mg), 3-bromopyridine **M1-7** (0.63 mmol, 100 mg), potassium phosphate (1.26 mmol, 268 mg) and CuI (0.08 mmol, 15 mg) were added to a microwave reaction bottle and dissolved in 6 mL DMF. After stirring for 5 minutes, N,N-dimethyl-1,2-cyclohexanediamine (0.08 mmol, 12 mg) was slowly added. The mixture was heated to 110°C under microwave, reacted for 1.5 hours, and cooled to room temperature. The reaction solution was added with 30 mL of water, extracted with ethyl acetate, dried over anhydrous sodium sulfate, and separated by flash column chromatography (PE/EA = 2/1) to obtain compound **M3-6** (100 mg, yield: 44%). LC-MS: $[M+H]^+$: 552.

The intermediate **M3-6** (100 mg) from the previous step was dissolved in 4 mL of dichloromethane, and 2 mL of trifluoroacetic acid was added, and the mixture was reacted at room temperature for 2 hours. The reaction was stopped, and the solvent was evaporated under reduced pressure. A saturated aqueous sodium bicarbonate solution was added to adjust the pH to 10. The mixture was extracted with dichloromethane, and separated by HPLC preparative chromatography (0.1% trifluoroacetic acid in water) to obtain the target compound **M3** (30 mg, yield: 37%). LC-MS: $[M+H]^+$: 468.
**M3:** $^1H$ NMR (400 MHz, DMSO-$d_6$) $\delta$ 12.71 (s, 1H), 8.83 (d, $J$ = 2.6 Hz, 1H), 8.68 (d, $J$ = 4.7 Hz, 1H), 8.27 (s, 1H), 8.09 - 8.02 (m, 1H), 7.63 (dd, $J$ = 8.2, 4.8 Hz, 2H), 6.72 - 6.76 (m, 2H), 4.49 (s, 2H), 4.17 (s, 2H), 3.20 (d, $J$ = 12.6 Hz, 2H), 2.34 (s, 3H), 2.24 (s, 3H), 1.86 - 1.89 (m, 2H), 1.76 - 1.81 (m, 2H).

**Example 2:** Preparation of Compounds **M4-M15**

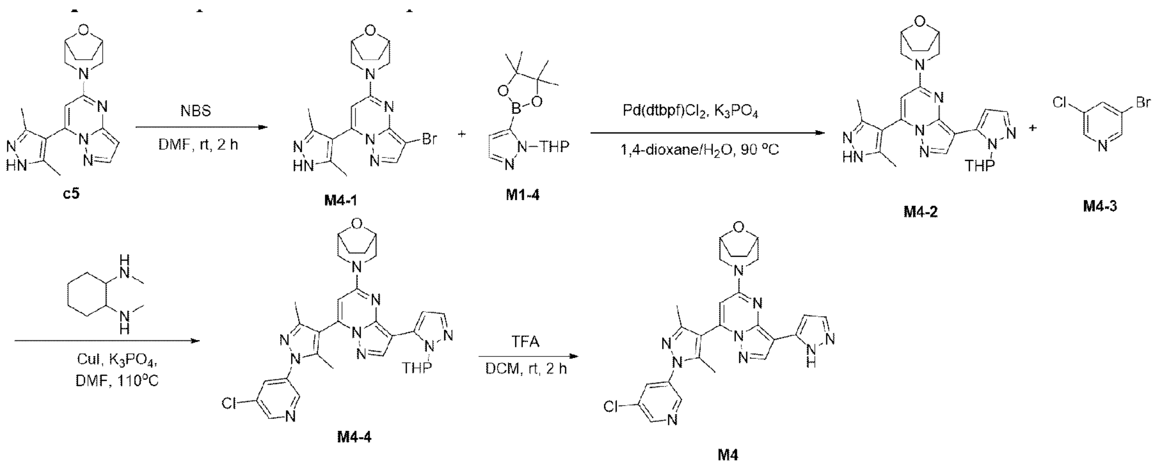


Step 1: Under nitrogen protection, intermediate **c5** of 3-(7-(3,5-dimethyl-1H-pyrazol-4-yl)pyrazolo[1,5-a]pyrimidin-5-yl)-8-oxa-3-azabicyclo[3.2.1]oc tane (1.85 mmol, 600.0 mg) was dissolved in 10 mL of DMF, and N-bromosuccinimide NBS (2.22 mmol, 395.1 mg) was slowly added. The mixture was reacted at room temperature for 2 hours, and the reaction was stopped. The reaction solution was added with 50 mL of water, extracted with ethyl acetate, washed with saturated brine, dried over anhydrous sodium sulfate, and concentrated. The crude product was separated and purified by column chromatography (DCM/MeOH = 10/1) to give compound **M4-1** of 3-(3-bromo-7-(3,5-dimethyl-1H-pyrazol-4-yl) pyrazolo[1,5-a]pyrimidin-5-yl)-8-oxa-3-azabicyclo[3.2.1]octane (600 mg, yield: 80%). LC-MS: $[M+H]^+$: 403.1.
$^1H$ NMR (400 MHz, DMSO-$d_6$) $\delta$ 12.67 (s, 1H), 7.95 (s, 1H), 6.58 (s, 1H), 4.46 (s, 2H), 4.09 (d, $J$ = 12.4 Hz, 2H), 3.18-3.07 (dd, $J$ = 12.4 Hz, 1.2 Hz, 2H), 2.17 (d, $J$ = 21.2 Hz, 7H), 1.90 - 1.81 (m, 2H), 1.77 - 1.67 (m, 2H).
Step 2: Under nitrogen protection, compound **M4-1** (1.24 mmol, 500 mg), potassium phosphate (3.72 mmol, 789.5 mg), compound **M1-4** (1.86 mmol, 571.3 mg) and catalyst Pd(dtbpf)$Cl_2$ (0.24 mmol, 155 mg) were dissolved in 10 mL of 1,4-dioxane, and 2 mL of water was added. After stirring for 5 minutes, the temperature was raised to 90° C and the reaction was continued for 16 hours, and cooled to room temperature. The reaction was stopped, and filtered. The mixture solution was added with 45 mL of water, extracted with ethyl acetate, washed with saturated brine, dried over anhydrous sodium sulfate, and concentrated. The crude product was separated and purified by column chromatography (DCM/MeOH = 10/1) to give compound **M4-2** of 3-(7-(3,5-dimethyl-1H-pyrazol-4-yl)-3-(1-(tetrahydro-2H-pyran-2-yl)-1H-pyrazol-5-yl)pyrazolo[1,5-a]pyrimidin-5-yl)-8-oxa-3-azabicyclo[3.2.1]octane (420 mg, yield: 71%). LC-MS: $[M+H]^+$: 475.5.
$^1H$ NMR (400 MHz, DMSO-$d_6$) $\delta$ 8.61 (s, 1H), 7.89 (d, $J$ = 8.0 Hz, 1H), 4.23 (q, $J$ = 7.2 Hz, 3H), 1.29 (t, $J$ = 6.8 Hz, 3H), 1.20 - 1.14 (m, 2H), 1.06 - 0.98 (m, 2H).
Step 3: Under nitrogen protection, compound **M4-2** (0.32 mmol, 150 mg), potassium phosphate (0.63 mmol, 131 mg), 3-bromo-5-chloropyridine **M4-3** (0.63 mmol, 122 mg) and ligand $N_1$,$N_2$-dimethyl 1,2-cyclohexanediamine (0.063 mmol, 8.99 mg) were dissolved in 5 mL of DMF, and catalyst CuI (0.03 mmol, 6.02 mg) was added. The temperature was raised to 110 ° C and the reaction was continued under stirring for 4 hours, and cooled to room temperature. The reaction was stopped. The mixture solution was added with 40 mL of water, extracted with ethyl acetate, washed with saturated brine, dried over anhydrous sodium sulfate, and concentrated. The crude product was separated and purified by column chromatography (DCM/MeOH = 15/1) to give compound **M4-4** of 3-(7-(1-(5-chloropyridin-3-yl)-3,5-dimethyl-1H-pyrazol-4-yl)-3-(1-(tetrahydro-2H-pyran-2-yl)-1H-pyrazol-5-yl)pyrazolo[1,5-a]pyrimidin-5-yl)-8-oxa-3-azabicyclo[3.2.1]octane (80 mg, yield: 44%). LC-MS: $[M+H]^+$: 586.3.

$^1$H NMR (400 MHz, DMSO-$d_6$) $\delta$ 8.61 (s, 1H), 7.89 (d, $J$ = 8.0 Hz, 1H), 4.23 (q, $J$ = 7.2 Hz, 3H), 1.29 (t, $J$ = 6.8 Hz, 3H), 1.20 - 1.14 (m, 2H), 1.06 - 0.98 (m, 2H).

Step 3: Under nitrogen protection, compound **M4-4** (0.14 mmol, 80 mg) was dissolved in 4 mL of a solvent mixture of dichloromethane and trifluoroacetic acid (v/v=3/1) and reacted at room temperature for 2 hours. A saturated aqueous sodium bicarbonate solution was added to the mixture solution to adjust the pH to about 9, extracted with ethyl acetate, washed with saturated brine, dried over anhydrous sodium sulfate, and concentrated. The crude product was separated and purified by HPLC preparative chromatography to give compound M4 (13.1 mg), LC-MS: $[M+H]^+$: 502.2.

$^1$H NMR (400 MHz, DMSO-$d_6$) $\delta$ 12.79 (br s, 1H), 8.83 (d, $J$ = 2.4 Hz, 1H), 8.75 (d, $J$ = 2.0 Hz, 1H), 8.27 (t, $J$ = 2.4 Hz, 2H), 7.59 (br s, 1H), 6.75 (br s, 1H), 6.69 (s, 1H), 4.50 (s, 2H), 4.15 (s, 2H), 3.20 (d, $J$ = 11.6 Hz, 2H), 2.38 (s, 3H), 2.24 (s, 3H), 1.90 - 1.74 (m, 4H).

Referring to the synthesis of compound **M4**, similar raw materials/intermediates were used to synthesize the target compounds **M5 to M15** below:

| **Raw materials/ Intermediates used** | **Structure of product** | **LC-MS/$^1$H NMR** |
|---|---|---|
| The structure of the raw material used in Step 3: 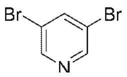 | 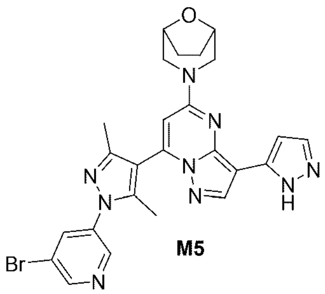 M5 | $[M+H]^+$ = 546.1. $^1$H NMR (400 MHz, DMSO-$d_6$) $\delta$ 12.65 (s, 1H), 8.86 (d, $J$ = 2.0 Hz, 1H), 8.82 (d, $J$ = 2.0 Hz, 1H), 8.37 (t, $J$ = 2.0 Hz, 1H), 8.27 (s, 1H), 7.60 (s, 1H), 6.76 (s, 1H), 6.69 (s, 1H), 4.50 (s, 2H), 4.16 (s, 2H), 3.20 (d, $J$ = 10.8 Hz, 2H), 2.38 (s, 3H), 2.24 (s, 3H), 1.90 - 1.83 (m, 2H), 1.82 - 1.75 (m, 2H). |
| The structure of the raw material used in Step 3: 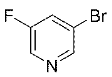 | 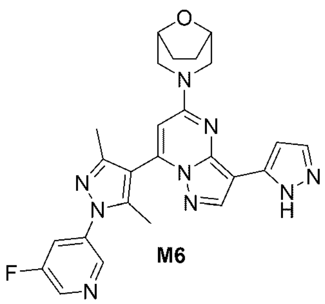 M6 | $[M+H]^+$ = 486.2. $^1$H NMR (400 MHz, DMSO-$d_6$) $\delta$ 8.76 (s, 1H), 8.72 (d, $J$ = 2.8 Hz, 1H), 8.27 (s, 1H), 8.14 - 8.09 (m, 1H), 7.62 (s, 1H), 6.77 (s, 1H), 6.70 (s, 1H), 4.50 (d, $J$ = 1.6 Hz, 2H), 4.16 (d, $J$ = 10.8 Hz, 2H), 3.20 (d, $J$ = 11.6 Hz, 2H), 2.38 (s, 3H), 2.24 (s, 3H), 1.92 - 1.75 (m, 4H). |
| The structure of the raw material used in Step 3: 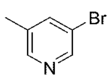 | 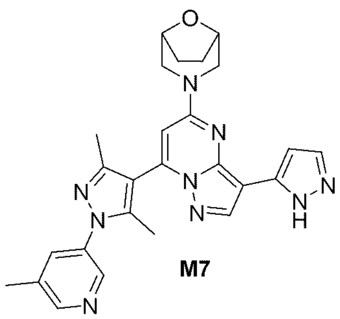 M7 | $[M+H]^+$ = 482.3. $^1$H NMR (400 MHz, DMSO-$d_6$) $\delta$ 12.70 (s, 1H), 8.62 (d, $J$ = 2.4 Hz, 1H), 8.52 (d, $J$ = 0.8 Hz, 1H), 8.27 (s, 1H), 7.88 (s, 1H), 7.60 (s, 1H), 6.76 (s, 1H), 6.70 (s, 1H), 4.50 (s, 2H), 4.17 (d, $J$ = 9.2 Hz, 2H), 3.20 (d, $J$ = 11.2 Hz, 2H), 2.43 (s, 3H), 2.33 (s, 3H), 2.23 (s, 3H), 1.90 - 1.75(m, 4H). |
| The structure of the raw material used in Step 3: 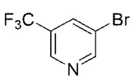 | 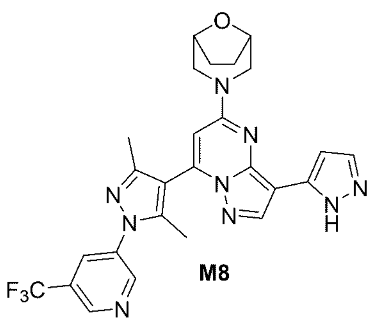 M8 | $[M+H]^+$ = 536.2. $^1$H NMR (400 MHz, $CDCl_3$) $\delta$ 9.03 (s, 1H), 8.92 (s, 1H), 8.19 (s, 2H), 7.64 (s, 1H), 6.52 (s, 1H), 6.26 (s, 1H), 4.60 (s, 2H), 4.00 (d, $J$ = 10.4 Hz, 2H), 3.45 - 3.39 (m, 2H), 2.41 (s, 3H), 2.36 (s, 3H), 2.11 - 2.04 (m, 2H), 1.88 (q, $J$ = 6.4 Hz, 2H). |

(continued)

| Raw materials/ Intermediates used | Structure of product | LC-MS/$^1$H NMR |
|---|---|---|
| The structure of the raw material used in Step 3: 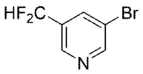 | 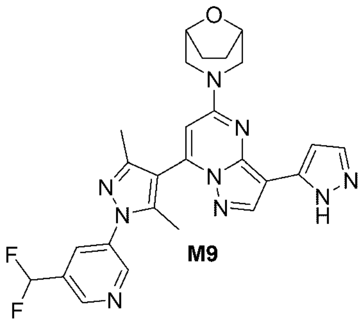 M9 | $[M+H]^+$ = 518.2. $^1$H NMR (400 MHz, $CDCl_3$) $\delta$ 8.95 (s, 1H), 8.80 (s, 1H), 8.24 (s, 1H), 8.08 (s, 1H), 7.68 (s, 1H), 6.81 (t, $J$ = 55.6 Hz, 1H), 6.58 (s, 1H), 6.26 (s, 1H), 4.61 (s, 2H), 4.01 (s, 2H), 3.42 (dd $J$ = 2 Hz, 12.4 Hz, 2H), 2.40 (s, 3H), 2.36 (s, 3H), 2.10 - 2.05 (m, 2H), 1.89 (d, $J$ = 6.8 Hz, 2H). |
| The structure of the raw material used in Step 3: 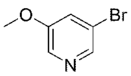 | 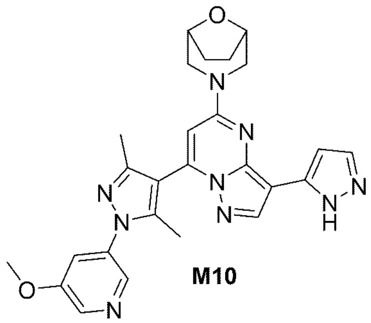 M10 | $[M+H]^+$ = 498.2. $^1$H NMR (400 MHz, $CDCl_3$) $\delta$ 8.38 (dd, $J$ = 2.8 Hz, 2.0 Hz, 2H), 8.20 (s, 1H), 7.65 (s, 1H), 7.44 (t, $J$ = 2.0 Hz, 1H), 6.53 (s, 1H), 6.26 (s, 1H), 4.60 (d, $J$ = 2.4 Hz, 2H), 4.01 (d, $J$ = 11.2 Hz, 2H), 3.93 (s, 3H), 3.41 (dd, $J$ = 12.4 Hz, 2.0 Hz, 2H), 2.37 (s, 3H), 2.35 (s, 3H), 2.12 - 2.01 (m, 2H), 1.89 (q, $J$ = 6.4 Hz, 2H). |
| The structure of the raw material used in Step 3: 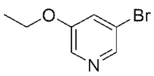 | 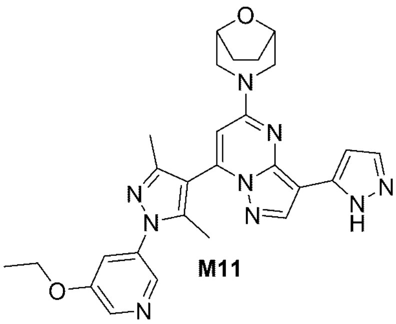 M11 | $[M+H]^+$ = 512.3. $^1$H NMR (400 MHz, $CDCl_3$) $\delta$ 8.36 (dd, $J$ = 4.4, 2.0 Hz, 2H), 8.21 (s, 1H), 7.66 (s, 1H), 7.42 (t, $J$ = 2.4 Hz, 1H), 6.54 (s, 1H), 6.25 (s, 1H), 4.60 (d, $J$ = 2.4 Hz, 2H), 4.15 (q, $J$ = 7.2 Hz, 2H), 4.01 (d, $J$ = 10.0 Hz, 2H), 3.41 (dd, $J$ = 12.0, 2.0 Hz, 2H), 2.37 (s, 3H), 2.34 (s, 3H), 2.09 - 2.01 (m, 2H), 1.89 (q, $J$= 6.4 Hz, 2H), 1.48 (t, $J$ = 7.2 Hz, 3H). |
| The structure of the raw material used in Step 3: 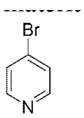 | 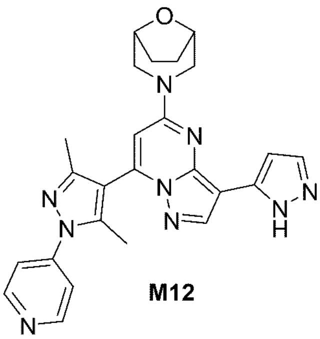 M12 | $[M+H]^+$ = 468.2. $^1$H NMR (400 MHz, $CDCl_3$) $\delta$ 7.27 (m, 1H), 7.23 - 7.18 (m, 2H), 7.13 (t, $J$ = 7.6 Hz, 1H), 6.97 - 6.91 (m, 1H), 6.83 (s, 1H), 6.76 (d, $J$ = 7.5 Hz, 1H), 6.71 (dd, $J$ = 8.0, 2.4 Hz, 1H), 4.32 - 4.27 (m, 1H), 3.80 (d, $J$ = 13.2 Hz, 1H), 3.38 (t, $J$ = 8.4 Hz, 1H), 3.04 (dd, $J$ = 19.6, 10.4 Hz, 2H), 2.67 (m, 1H), 2.33 (dd, $J$ = 10.4, 4.8 Hz, 1H), 1.79 - 1.72 (m, 1H). |

(continued)

| Raw materials/ Intermediates used | Structure of product | LC-MS/$^1$H NMR |
|---|---|---|
| The structure of the raw material used in Step 3: 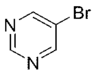 | 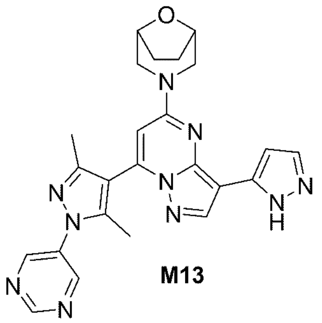 M13 | $[M+H]^+$ = 469.2. $^1$H NMR (400 MHz, DMSO-$d_6$) $\delta$ 12.70 (s, 1H), 9.29 (s, 1H), 9.14 (s, 2H), 8.27 (s, 1H), 7.60 (s, 1H), 6.76 (s, 1H), 6.70 (s, 1H), 4.50 (s, 2H), 4.15 (s, 2H), 3.20 (d, $J$ = 11.2 Hz, 2H), 2.40 (s, 3H), 2.25 (s, 3H), 1.90 - 1.85 (m, 2H), 1.78 (dd, $J_1$ = 5.2, $J_2$ = 12.4, 6.9 Hz, 2H). |
| The structure of the raw material used in Step 3: 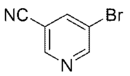 | 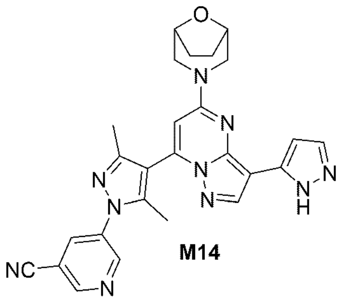 M14 | $[M+H]^+$ = 493.2. $^1$H NMR (400 MHz, DMSO-$d_6$) $\delta$ 12.56 (s, 1H), 9.14 (d, $J$ = 2. 0 Hz, 1H), 9.11 (d, $J$ = 1.2 Hz, 1H), 8.65 (t, $J$ = 2.0 Hz, 1H), 8.27 (s, 1H), 7.62 (s, 1H), 6.78 (s, 1H), 6.69 (s, 1H), 4.50 (s, 2 H), 4.16 (d, $J$ = 8.8 Hz, 2H), 3. 20 (d, $J$ = 11.6 Hz, 2H), 2.40 (s, 3H), 2.25 (s, 3H), 1.91 - 1.8 4 (m, 2H), 1.78 (dd, $J$ = 14.4, 7.2 Hz, 2H). |
| The structure of the raw material used in Step 3: 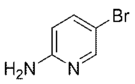 | 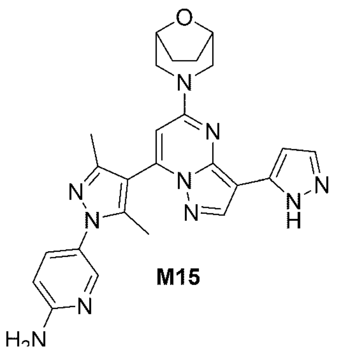 M15 | $[M+H]^+$ = 483.3. $^1$H NMR (400 MHz, $CDCl_3$) $\delta$ 8.16 (s, 2H), 7.70 - 7.59 (m, 2H), 6.66 (d, $J$ = 9.2 Hz, 1H), 6.49 (s, 1H), 6.23 (s, 1H), 5.03 (s, 2H), 4.59 (s, 2H), 3.98 (s, 2H), 3.40 (d, $J$ = 10.4 Hz, 2H), 2.33 (d, $J$ = 4.4 Hz, 3H), 2.28 (s, 3H), 2.06 (d, $J$ = 8.8 Hz, 2H), 1.88 (d, $J$ = 6.8 Hz, 2H). |

**Example 3:** Preparation of Compounds **M16-M18**

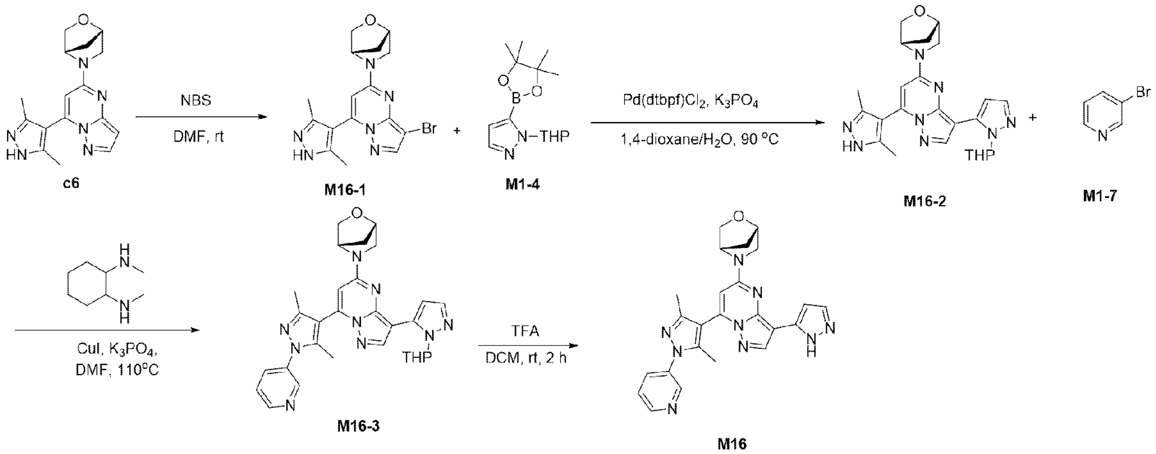


c6, M16-1, M1-4, M16-2, M1-7, M16-3, M16

Step 1: Under nitrogen protection, intermediate **c6** of (*1S,4S*)-5-(7-(3,5-dimethyl-1H-pyrazol-4-yl)pyrazolo[1,5-a]pyrimidin-5-yl)-2-oxa-5-azabicyclo[2.2.1]heptane (0.73 mmol, 300.0 mg) was dissolved in 5 mL of DMF, and N-bromosuccinimide NBS (0.89 mmol, 156.1 mg) was slowly added. The mixture was allowed to react at room temperature for 2 hours, and the reaction was stopped. The reaction solution was added with 30 mL of water, extracted with dichloromethane, washed with saturated brine, dried over anhydrous sodium sulfate, and concentrated. The crude product was separated and purified by flash reverse column chromatography (acetonitrile/water) to obtain compound **M16-1** of (*1S*,*4S*)-5-(3-bromo-7-(3,5-dimethyl-1H-pyrazol-4-yl)pyrazolo[1,5-a]pyrimidin-5-yl)-2-oxa-5-azabicyclo[2.2.1]heptane (5.0 mg, yield: 6%). LC-MS: $[M+H]^+$: 309.9. The reaction was scaled up repeatedly to afford **M16-1** (2.0 g).

Step 2: Under nitrogen protection, compound **M16-1** (0.59 mmol, 230 mg), potassium phosphate (1.77 mmol, 376 mg), compound **M1-4** (1.18 mmol, 329 mg) and catalyst Pd(dtbpf)$Cl_2$ (0.12 mmol, 76 mg) were dissolved in 4 mL of 1,4-dioxane, and 0.8 mL of water was added. After stirring for 5 minutes, the mixture was heated to 90 ° C and continued to stir for 16 hours, and cooled to room temperature. The reaction was stopped, and filtered. The mixture solution was added with 30 mL of water, extracted with ethyl acetate, washed with saturated brine, dried over anhydrous sodium sulfate, and concentrated. The crude product was separated and purified by column chromatography (DCM/MeOH = 50/1) to give compound **M16-2** of (*1S*,*4S*)-5-(7-(3,5-dimethyl-1H-pyrazol-4-yl)-3-(1-(tetrahydro-2H-pyran-2-yl)-1H-pyrazol-5-yl)pyrazolo[1,5-a]pyrimidin-5-yl)-2-oxa-5-azabicyclo[2.2.1]heptane (150 mg, yield: 55%). LC-MS: $[M+H]^+$: 461.1.

Step 3: Under nitrogen protection, compound **M16-2** (0.33 mmol, 150 mg), potassium phosphate (0.65 mmol, 138 mg), 3-bromopyridine **M1-7** (0.65 mmol, 103 mg) and ligand $N_1$,$N_2$-dimethyl 1,2-cyclohexanediamine (0.65 mmol, 142 mg) were dissolved in 5 mL of DMF, and catalyst CuI (0.33 mmol, 62 mg) was added. The mixture was heated to 110 ° C, continued to stir for 16 hours, and cooled to room temperature to stop the reaction. The mixture solution was added with 40 mL of water, extracted with ethyl acetate, washed with saturated brine, dried over anhydrous sodium sulfate, and concentrated to obtain a crude compound **M16-3**. LC-MS: $[M+H]^+$: 538.3.

Step 4: Under nitrogen protection, the crude compound **M16-3** obtained in Step 3 was dissolved in 4 mL of a solvent mixture of dichloromethane and trifluoroacetic acid (v/v=3/1), and reacted at room temperature for 2 hours. A saturated aqueous sodium bicarbonate solution was added to the mixture solution to adjust the pH to about 9, extracted with ethyl acetate, washed with saturated brine, dried over anhydrous sodium sulfate, and concentrated. The crude product was separated and purified by HPLC preparative chromatography to give compound **M16** (13.8 mg). LC-MS: $[M+H]^+$: 454.2.

$^1$H NMR (400 MHz, DMSO-$d_6$) $\delta$ 12.64 (s, 1H), 8.83 (d, *J* = 2.4 Hz, 1H), 8.67 (d, *J* = 4.8 Hz, 1H), 8.25 (s, 1H), 8.08 - 8.02 (m, 1H), 7.63 (dd, *J* = 8.0 Hz, 4.8 Hz, 2H), 6.64 (br s, 2H), 5.06 (s, 1H), 4.75 (s, 1H), 3.83 (d, *J* = 15.6 Hz, 2H), 3.63 (d, *J* = 10.0 Hz, 2H), 2.34 (s, 3H), 2.24 (s, 3H), 1.97 (dd, *J* = 25.2, 9.2 Hz, 2H).

Referring to the synthesis of compound **M16**, similar raw materials/intermediates were used to synthesize the target compounds **M17 to M18** below:

| **Raw materials/ Intermediates used** | **Structure of product** | **LC-MS/$^1$H NMR** |
|---|---|---|
| The structure of the intermediate used in Step 1: **c7**. | 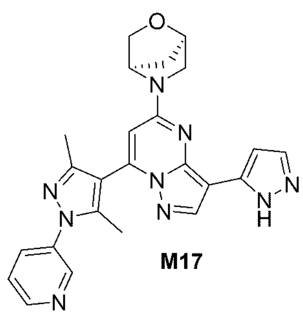 **M17** | $[M+H]^+$ = 454.2.<br>$^1$H NMR (400 MHz, $CDCl_3$) $\delta$ 8.82 (d, *J* = 2.0 Hz, 1H), 8.67 (dd, *J* = 4.8 Hz, 1.2 Hz, 1H), 8.17 (s, 1H), 7.94 - 7.88 (m, 1H), 7.66 (s, 1H), 7.48 (dd, $J_1$ = 8.0 Hz, 4.8 Hz, 1H), 6.52 (s, 1H), 6.07 (s, 1H), 5.35 (s, 1H), 4.82 (s, 1H), 4.03 (s, 2H), 3.66 (s, 2H), 2.37 (s, 3H), 2.35 (s, 3H), 2.15 - 2.04 (m, 2H). |

(continued)

| Raw materials/ Intermediates used | Structure of product | LC-MS/$^1$H NMR |
|---|---|---|
| The structure of the intermediate used in Step 1: **c8**. | 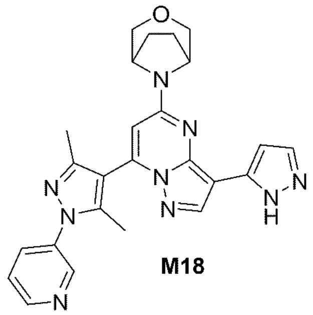 **M18** | $[M+H]^+$ = 468.2. $^1$H NMR (400 MHz, DMSO-$d_6$) $\delta$ 8.82 (d, $J$ = 2.4 Hz, 1H), 8.67 (dd, $J$ = 4.8 Hz, 1.6 Hz, 1H), 8.17 (s, 1H), 7.93 - 7.89 (m, 1H), 7.63 (s, 1H), 7.48 (dd, $J$ = 8.0 Hz, 4.8 Hz, 1H), 6.50 (s, 1H), 6.21 (s, 1H), 4.62 (s, 2H), 3.90 (d, $J$ = 11.2 Hz, 2H), 3.74 (d, J = 10.8 Hz, 2H), 2.27 - 2.11 (m, 4H). |

**Example 4:** Preparation of Compounds **M1a** and **M1b**

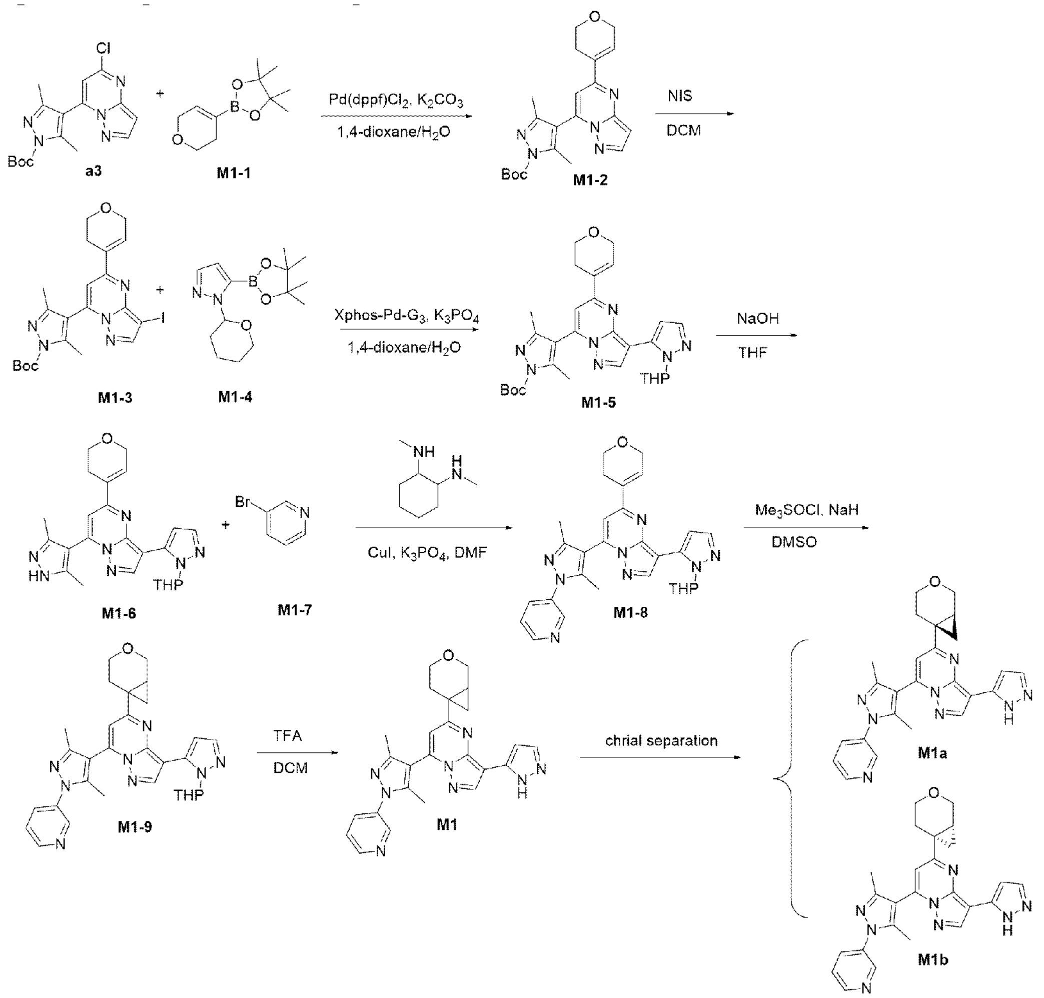


Under nitrogen protection, intermediate **a3** (3.75 mmol, 1.3 g) and raw material **M1-1** (5.63 mmol, 1.18 g) were dissolved in 30 mL of a mixture of 1,4-dioxane and water (v/v: 9/1), potassium carbonate (11.25 mmol, 1.55 g) and Pd(dppf) $Cl_2$ (0.4 mmol, 292 mg) were added. The reaction was heated at 100 °C for 10 hours. The reaction was stopped, and

filtered. 80 mL of water was added to the system. The mixture was extracted with ethyl acetate, dried over anhydrous sodium sulfate, and separated by flash column chromatography (PE/EA = 2/1) to obtain compound **M1-2** (1.1 g, yield: 75%). LC-MS: $[M+H]^+$: 396.

Under an ice bath, intermediate **M1-2** (2.78 mmol, 1.1 g) was dissolved in 25 mL of anhydrous dichloromethane, N-iodosuccinimide NIS (3.06 mmol, 688 mg) was slowly added in portions, and stirring was continued for 2 hours. The reaction was stopped. 30 mL of a saturated aqueous ammonium chloride solution was added. The mixture was extracted with dichloromethane, dried over anhydrous sodium sulfate and separated by flash column chromatography (PE/EA = 2/1) to obtain compound **M1-3** (1.4 g, yield: 97%). LC-MS: $[M+H]^+$: 522.

Under nitrogen protection, intermediate **M1-3** (2.69 mmol, 1.4 g) and raw material **M1-4** (4.03 mmol, 1.1 g) were dissolved in 20 mL of a mixture of 1,4-dioxane and water (v/v: 9/1), and potassium phosphate (5.38 mmol, 1.1 g) and Xphos-Pd-G3 (0.27 mmol, 229 mg) were added. The reaction was heated under microwave at 95 °C for 2 hours. The reaction was stopped and filtered. 60 mL of water was added to the system. The mixture was extracted with ethyl acetate, dried over anhydrous sodium sulfate, and separated by flash column chromatography (PE/EA = 4/1) to obtain compound **M1-5** (700 mg, yield: 48%). LC-MS: $[M+H]^+$: 546.

Intermediate **M1-5** (700 mg) was dissolved in 15 mL of tetrahydrofuran, and 6 mL of 1N NaOH aqueous solution was added. The mixture was heated to 70 °C to react for 1 hour. The reaction was stopped, and the solvent was evaporated under reduced pressure. 30 mL of water was added, and the mixture was extracted with dichloromethane, and separated by flash column chromatography (PE/EA = 1/1) to obtain compound **M1-6** (530 mg, yield: 93%). LC-MS: $[M+H]^+$: 446.

Under nitrogen protection, intermediate **M1-6** (1.19 mmol, 530 mg), 3-bromopyridine **M1-7** (1.78 mmol, 282 mg), potassium phosphate (2.38 mmol, 505 mg) and CuI (0.12 mmol, 23 mg) were added to a microwave reaction bottle and dissolved in 10 mL of DMF. After stirring for 5 minutes, N,N-dimethyl-1,2-cyclohexanediamine (0.24 mmol, 34 mg) was slowly added. The mixture was heated to 110 °C under microwave and reacted for 1.5 hours, cooled to room temperature, and filtered. The reaction solution was added with 50 mL of water, extracted with ethyl acetate, dried over anhydrous sodium sulfate, and separated by flash column chromatography to obtain compound **M1-8** (240 mg, yield: 39%). LC-MS: $[M+H]^+$: 523.

Under nitrogen protection, NaH (1.66 mmol, 60%, 40 mg) and trimethyl sulfoxide chloride (1.0 mmol, 129 mg) were added to the reaction flask, and 1 mL of anhydrous DMSO was slowly added to the system and stirred for 20 minutes. Intermediate **M1-8** (0.46 mmol, 240 mg) was dissolved in 1 mL of DMSO, and the mixture was slowly injected into the reaction solution. The solution was heated to 65°C and reacted for 4 hours, and the reaction was stopped. The reaction solution was added with 20 mL of water, and extracted with ethyl acetate to obtain 200 mg of the crude intermediate **M1-9**. LC-MS: $[M+H]^+$: 537.

The crude intermediate **M1-9** (200 mg) was dissolved in 4 mL of dichloromethane, and 2 mL of trifluoroacetic acid was added, and the mixture was reacted at room temperature for 2 hours. The reaction was stopped, and the solvent was evaporated under reduced pressure. A saturated aqueous sodium bicarbonate solution was added to adjust the pH to 10. The mixture was extracted with dichloromethane, and separated by HPLC preparative chromatography to obtain compound **M1** (70 mg, two-step yields: 34%). LC-MS: $[M+H]^+$: 453.

Compound **M1** was separated by chiral preparative chromatography to obtain target compounds **M1a** and **M1b**.

**M1b:** $^1$H NMR (400 MHz, DMSO-$d_6$) $\delta$ 12.91 (d, $J$ = 111.8 Hz, 1H), 8.85 (d, $J$ = 2.5 Hz, 1H), 8.68 (d, $J$ = 4.7 Hz, 1H), 8.50 (s, 1H), 8.10 - 8.03 (m, 1H), 7.77 (s, 1H), 7.64 (dd, $J$ = 8.2, 4.8 Hz, 1H), 6.92 (s, 2H), 3.90 - 3.97 (m, 2H), 3.65 - 3.70 (m, 1H), 3.38 - 3.41 (m, 1H), 2.91 - 2.97 (m, 1H), 2.34 (s, 3H), 2.24 (s, 3H), 1.99 - 2.01 (m, 1H), 1.91 (s, 1H), 1.55 (dd, $J$ = 9.3, 4.2 Hz, 1H), 1.23 (d, $J$ = 5.5 Hz, 1H).

**Example 5:** Preparation of Compounds **M2a** and **M2b**

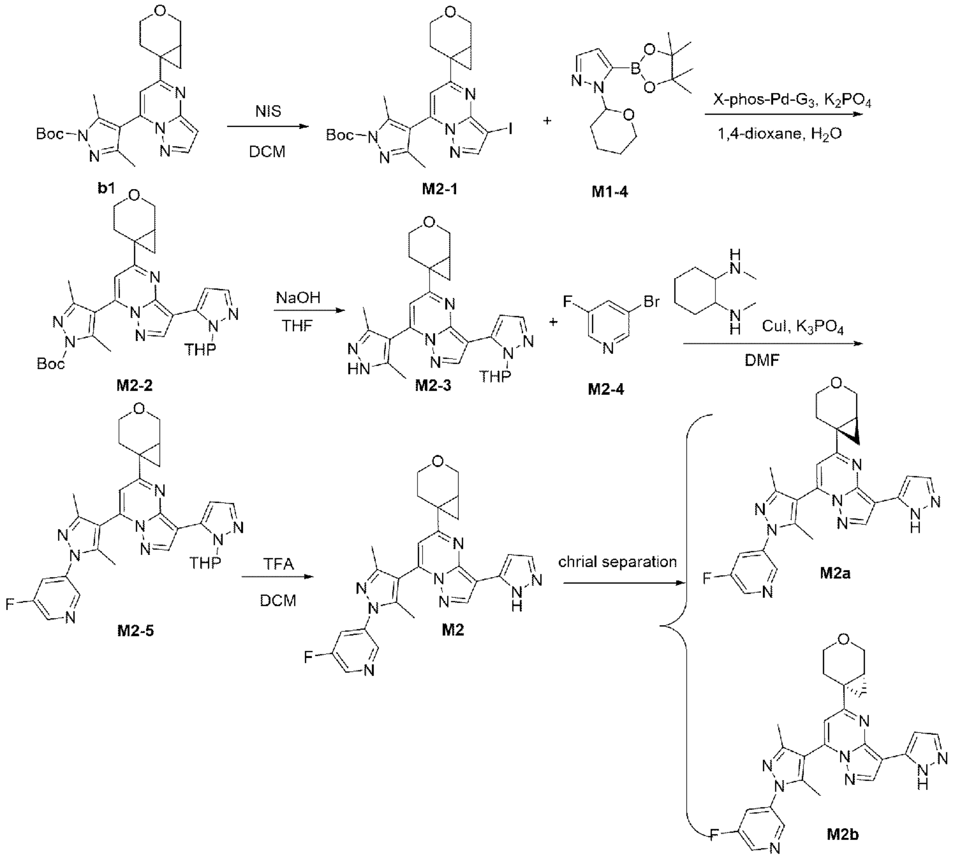


Under an ice bath, intermediate **b1** (3.18 mmol, 1.3 g) was dissolved in 25 mL of anhydrous dichloromethane, N-iodosuccinimide NIS (3.50 mmol, 787 mg) was slowly added in portions and stirring was continued for 2 hours. The reaction was stopped. 35 mL of a saturated aqueous ammonium chloride solution was added. The mixture was extracted with dichloromethane, dried over anhydrous sodium sulfate and separated by flash column chromatography (PE/EA = 2/1) to obtain compound **M2-1** (1.3 g, yield: 77%). LC-MS: $[M+H]^+$: 536.

Under nitrogen protection, intermediate **M2-1** (2.43 mmol, 1.3 g) and raw material **M1-4** (3.65 mmol, 1.01 g) were dissolved in 20 mL of a mixture of 1,4-dioxane and water (v/v: 9/1), and potassium phosphate (4.86 mmol, 1.03 g) and Xphos-Pd-G3 (0.24 mmol, 203 mg) were added. The reaction was heated under microwave at 95 °C 2 hours. The reaction was stopped and filtered. 60 mL of water was added to the system. The mixture was extracted with ethyl acetate, dried over anhydrous sodium sulfate, and separated by flash column chromatography (PE/EA = 4/1) to obtain compound **M2-2** (1.3 g, yield: 96%). LC-MS: $[M+H]^+$: 560.

Intermediate **M2-2** (1300 mg) was dissolved in 20 mL of tetrahydrofuran, and 8 mL of 1N NaOH aqueous solution was added. The mixture was heated to 70 °C to react for 1 hour. The reaction was stopped, and the solvent was evaporated under reduced pressure. 40 mL of water was added. The mixture was extracted with dichloromethane, and separated by flash column chromatography (PE/EA = 1/1) to obtain compound **M2-3** (1.0 g, yield: 94%). LC-MS: $[M+H]^+$: 460.

Under nitrogen protection, intermediate **M2-3** (0.65 mmol, 300 mg), 3-bromo-5-fluoro-pyridine **M2-4** (0.98 mmol, 172 mg), potassium phosphate (1.30 mmol, 276 mg) and CuI (0.06 mmol, 12 mg) were added to a microwave reaction bottle and dissolved in 8 mL DMF. After stirring for 5 minutes, N,N-dimethyl-1,2-cyclohexanediamine (0.12 mmol, 17 mg) was slowly added. The mixture was heated to 110° C under microwave for 1.5 hours, and then the reaction was stopped and filtered. The reaction solution was added with 30 mL of water, extracted with ethyl acetate, dried over anhydrous sodium sulfate, and separated by flash column chromatography (PE/EA = 3/1) to obtain compound **M2-5** (200 mg, yield: 56%). LC-MS: $[M+H]^+$: 555.

Intermediate **M2-5** (0.36 mmol, 200 mg) was dissolved in 4 mL of dichloromethane, 2 mL of trifluoroacetic acid was added, and the mixture was reacted at room temperature for 2 hours. The reaction was stopped, and the solvent was evaporated under reduced pressure. A saturated aqueous sodium bicarbonate solution was added to adjust the pH to 10. The mixture was extracted with dichloromethane, and separated by HPLC preparative chromatography to obtain compound **M2** (80 mg, yield: 48%). LC-MS: $[M+H]^+$: 471.

Compound **M2** was separated by chiral preparative chromatography to obtain target compounds **M2a** and **M2b**.

**M2b:** $^1$H NMR (400 MHz, DMSO-$d_6$) $\delta$ 12.91 (d, $J$ = 117.3 Hz, 1H), 8.77 (s, 1H), 8.73 (d, $J$ = 2.6 Hz, 1H), 8.49 (s, 1H), 8.14 (dd, $J$ = 9.6, 2.7 Hz, 1H), 7.69 (d, $J$ = 79.0 Hz, 1H), 6.90 (s, 2H), 3.90 - 3.97 (m, 2H), 3.65 - 3.70 (m, 1H), 3.35 - 3.41

(m, 1H), 2.94 (dt, $J$ = 14.4, 4.6 Hz, 1H), 2.38 (s, 3H), 2.24 (s, 3H), 2.01 - 2.08 (m, 1H), 1.91 (s, 1H), 1.54 (dd, $J$ = 9.2, 4.2 Hz, 1H), 1.22 - 1.26 (m, 1H).

**Example 6:** Preparation of Compound **M19**

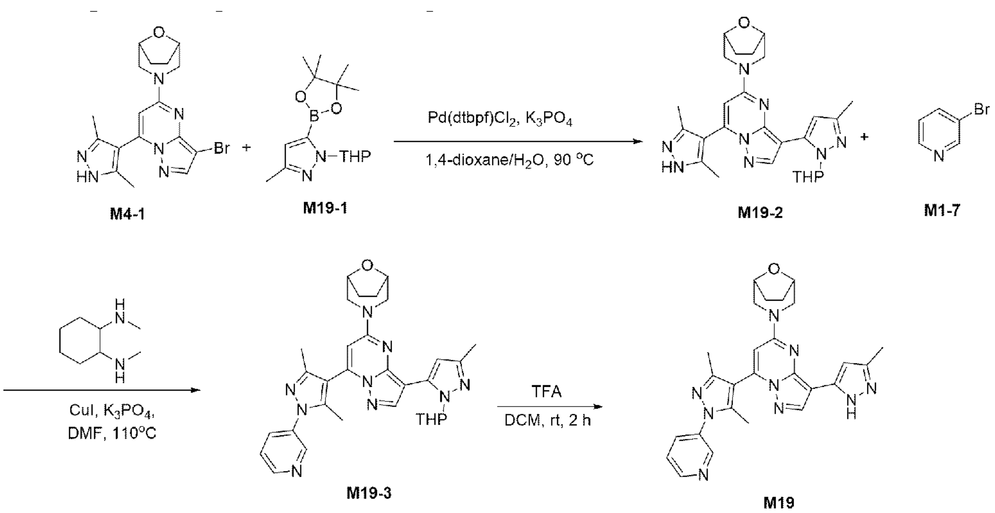

M4-1 M19-1 M19-2 M1-7
M19-3 M19

Step 1: Under nitrogen protection, compound **M4-1** of 3-(3-bromo-7-(3,5-dimethyl-1H-pyrazol-4-yl)pyrazolo[1,5-a]pyrimidin-5-yl)-8-oxa-3-azabicyclo[3.2.1]octane (1.24 mmol, 500 mg), potassium phosphate (3.72 mmol, 789.5 mg), compound **M19-1** (1.49 mmol, 434.7 mg) and catalyst Pd(dtbpf)$Cl_2$ (0.12 mmol, 80 mg) were dissolved in 10 mL of 1,4-dioxane, and 2 mL of water was added. The mixture was stirred for 5 minutes, heated to 90 °C and continued to stir for 16 hours, and cooled to room temperature. The reaction was stopped and filtered. The mixture solution was added with 40 mL of water, extracted with ethyl acetate, washed with saturated brine, dried over anhydrous sodium sulfate, and concentrated. The crude product was separated and purified by column chromatography (DCM/MeOH = 10/1) to give compound **M19-2** of 3-(7-(3,5-dimethyl-1H-pyrazol-4-yl)-3-(3-methyl-1-(tetrahydro-2H-pyran-2-yl)-1H-pyrazol-5-yl)pyrazolo[1,5-a]pyrimidin-5-yl)-8-oxa-3-azabicyclo[3.2.1]octane (460 mg, yield: 76%). LC-MS: $[M+H]^+$: 489.3.

Step 2: Under nitrogen protection, compound **M19-2** (0.41 mmol, 200 mg), potassium phosphate (0.82 mmol, 174 mg), 3-bromo-pyridine **M1-7** (0.82 mmol, 130 mg) and ligand $N_1$,$N_2$-dimethyl 1,2-cyclohexanediamine (0.41 mmol, 58 mg) were dissolved in 5 mL of DMF, and catalyst CuI (0.20 mmol, 39 mg) was added. The mixture was heated to 110 °C and stirred for 16 hours, and cooled to room temperature to stop the reaction. The mixture solution was added with 40 mL of water, extracted with ethyl acetate, washed with saturated brine, dried over anhydrous sodium sulfate, and concentrated to obtain a crude compound **M19-3**. LC-MS: $[M+H]^+$: 566.3.

Step 3: Under nitrogen protection, the crude **M19-3** obtained in Step 2 was dissolved in 6 mL of a solvent mixture of dichloromethane and trifluoroacetic acid (v/v=3/1), reacted at room temperature for 2 hours, and then the reaction was stopped. A saturated sodium bicarbonate aqueous solution was added to the mixture solution to adjust the pH to about 9. The mixture solution was extracted with ethyl acetate, washed with saturated brine, dried over anhydrous sodium sulfate, and concentrated. The crude product was separated and purified by HPLC preparative chromatography to give compound **M19** (79.5 mg). LC-MS: $[M+H]^+$: 482.3.

$^1$H NMR (400 MHz, DMSO-$d_6$) $\delta$ 12.30 (s, 1H), 8.83 (d, $J$ = 2.4 Hz, 1H), 8.67 (dd, $J$ = 4.8 Hz, 1.2 Hz, 1H), 8.23 (s, 1H), 8.07 - 8.02 (m, 1H), 7.65 - 7.60 (m, 1H), 6.70 (s, 1H), 6.51 (s, 1H), 4.50 (d, $J$ = 2.0 Hz, 2H), 4.16 (d, $J$ = 12.4 Hz, 2H), 3.19 (d, $J$ = 11.2 Hz, 2H), 2.33 (s, 3H), 2.23 (s, 6H), 1.92 - 1.73 (m, 4H).

**Example 7**: Preparation of Compound **M20**

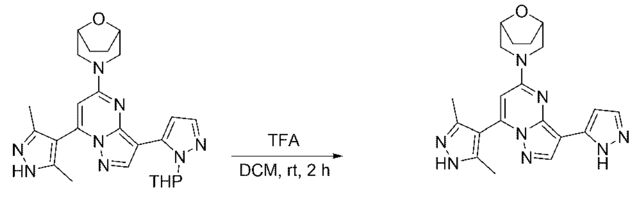


M4-2 M20

Under nitrogen protection, compound **M4-2** of 3-(7-(3,5-dimethyl-1H-pyrazol-4-yl)-3-(1-(tetrahydro-2H-pyran-2-yl)-1H-pyrazol-5-yl)pyrazolo [1,5-a]pyrimidin-5-yl)-8-oxa-3-azabicyclo[3.2.1]octane (0.11 mmol, 50 mg) was dissolved in 4 mL of a solvent mixture of dichloromethane and trifluoroacetic acid (v/v=3/1), and reacted at room temperature for 2 hours, and then the reaction was stopped. A saturated aqueous sodium bicarbonate solution was added to the mixture solution to adjust the pH to about 9. The mixture solution was extracted with ethyl acetate, washed with saturated brine, dried over anhydrous sodium sulfate, and concentrated. The crude product was separated and purified by HPLC preparative chromatography to give compound **M20** (18.0 mg). LC-MS: $[M+H]^+$: 391.2.

$^1$H NMR (400 MHz, DMSO-$d_6$) $\delta$ 12.67 (s, 2H), 8.23 (s, 1H), 7.58 (s, 1H), 6.73 (s, 1H), 6.56 (s, 1H), 4.48 (s, 2H), 4.14 (s, 2H), 3.16 (d, $J$ = 10.8 Hz, 2H), 2.20 (s, 6H), 1.90 - 1.73 (m, 4H).

**Example 8:** Preparation of Compounds **M21-M22**

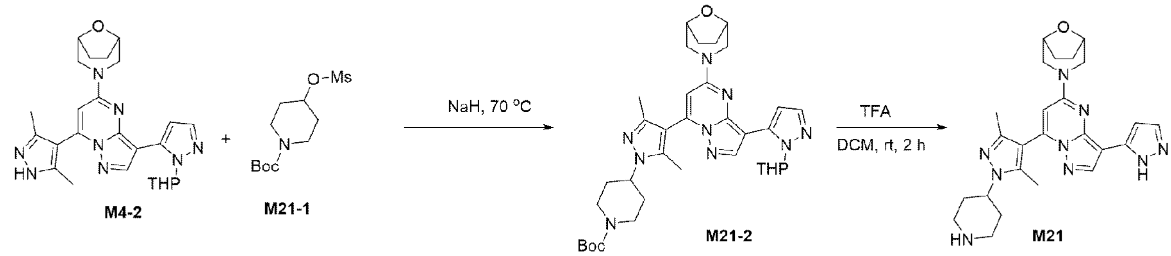


M4-2 M21-1 M21-2 M21

Step 1: Under nitrogen protection, compound **M4-2** of 3-(7-(3,5-dimethyl-1H-pyrazol-4-yl)-3-(1-(tetrahydro-2H-pyran-2-yl)-1H-pyrazol-5-yl)pyrazolo [1,5-a]pyrimidin-5-yl)-8-oxa-3-azabicyclo[3.2.1]octane (0.32 mmol, 150 mg) was dissolved in 5 mL of anhydrous DMF, and NaH (0.47 mmol, 18.96 mg) was added. After stirring for 5 minutes, raw material **M21-1** (0.47 mmol, 132.4 mg) was added. The mixture was heated to 70 °C and continued to stir for 16 hours, and cooled to room temperature. The reaction was stopped, and filtered. The mixture solution was added with 40 mL of water, extracted with ethyl acetate, washed with saturated brine, dried over anhydrous sodium sulfate, and concentrated to obtain a crude compound **M21-2**. LC-MS: $[M+H]^+$: 658.5.

Step 2: Under nitrogen protection, the crude product **M21-2** obtained in Step 1 was dissolved in 4 mL of a solvent mixture of dichloromethane and trifluoroacetic acid (v/v=3/1), reacted at room temperature for 2 hours, and then the reaction was stopped. A saturated aqueous sodium bicarbonate solution was added to the mixture solution to adjust the pH to about 9. The mixture solution was extracted with ethyl acetate, washed with saturated brine, dried over anhydrous sodium sulfate, and concentrated. The crude product was separated and purified by HPLC preparative chromatography to give compound **M21** (38.89 mg). LC-MS: $[M+H]^+$: 474.3.

$^1$H NMR (400 MHz, $CDCl_3$) $\delta$ 8.34 (s, 1H), 8.23 (s, 1H), 7.59 (s, 1H), 6.74 (s, 1H), 6.57 (s, 1H), 4.48 (s, 2H), 4.44 - 4.35 (m, 1H), 4.15 (d, $J$ = 12.0 Hz, 2H), 3.25 (d, $J$ = 11.2 Hz, 2H), 3.16 (d, $J$ = 11.6 Hz, 2H), 2.87 (t, $J$ = 11.6 Hz, 2H), 2.25 (s, 3H), 2.15 - 2.03 (m, 5H), 1.94 - 1.82 (m, 4H), 1.80 - 1.72 (m, 2H).

Referring to the synthesis of compound **M21**, similar raw materials/intermediates were used to synthesize the target compound **M22** below:

| Raw materials/ Intermediates used | Structure of product | LC-MS/$^1$H NMR |
|---|---|---|
| The structure of the raw material used in Step 1: 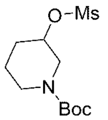 | 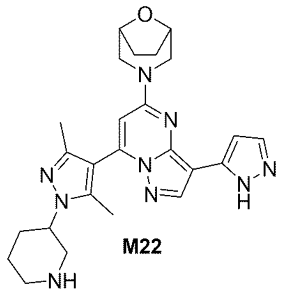 M22 | $[M+H]^+$ = 474.3. $^1$H NMR (400 MHz, $CDCl_3$) $\delta$ 7.27 (m, 1H), 7.23 - 7.18 (m, 2H), 7.13 (t, *J* = 7.6 Hz, 1H), 6.97 - 6.91 (m, 1H), 6.83 (s, 1H), 6.76 (d, *J* = 7.5 Hz, 1H), 6.71 (dd, *J* = 8.0, 2.4 Hz, 1H), 4.32 - 4.27 (m, 1H), 3.80 (d, *J* = 13.2 Hz, 1H), 3.38 (t, *J* = 8.4 Hz, 1H), 3.04 (dd, *J* = 19.6, 10.4 Hz, 2H), 2.67 (m, 1H), 2.33 (dd, *J* = 10.4, 4.8 Hz, 1H), 1.79 - 1.72 (m, 1H). |

**Example 9: ATR kinase activity assay**

ATR kinase activity assay was performed by detecting the phosphorylation of the downstream substrate p53 protein by ATR kinase. GST-tagged full-length P53 protein was purchased from Sigma (Cat. No.: 14-865), and Anti-phospho-p53(ser15)-K antibody and Anti-GST-d2 antibody were both purchased from Cisbio (Cat. Nos.: 61GSTDLA; 61P08KAE). The amount of phosphorylated P53 protein was determined using a time-resolved fluorescence system. Before the starting of the experiment, the following working solutions were prepared as needed: 1× reaction buffer (20mM HEPES PH8.0, 1% glycerol, 0.01% Brij-35), dilution buffer (20mM HEPES PH8.0, 1% glycerol, 0.01% Brij-35, 5mM DTT and 1% BSA), stop buffer (20mM HEPES PH8.0, 1% glycerol, 0.01% Brij-35, 250mM EDTA), detection buffer (50mM HEPES PH7.0, 150mM NaCl, 267mM KF, 0.1% sodium cholate, 0.01% Tween-20, 0.0125% sodium azide). The clinically investigational drug **AZD6738** (purchased from Selleck) was used as a positive control.

The specific steps of the assay are as follows:

4× gradient dilution compound solutions were prepared with the 1× reaction buffer to obtain 9 different compound concentrations, and 2.5 μL of the 4× gradient dilution compound solutions are added to a 384-well assay plate (784075, Greiner). 4× p53 substrate working solution (40 nM) was prepared with the 1× reaction buffer and 2.5 μL of the 4× p53 substrate working solution was added to the 384-well assay plate. 4× ATR/ATRIP working solution (12.8 ng/μL) was prepared with the dilution buffer and 2.5 μL of the 4× ATR/ATRIP working solution was added to the 384-well assay plate. 4×ATP working solution (2 mM) was prepared with deionized water, and 2.5 μL of the 4×ATP working solution was added to the 384-well assay plate. The plate was incubated at room temperature for 30 minutes in the dark. 5 μL of the stop solution was added to the 384 assay plate. Finally, 5 μL of the detection mixture (0.09 ng/μL of Anti-phospho-p53(ser15)-K and 6 ng/μL of Anti-GST-d2) to the 384-well assay plate. After incubation at room temperature overnight, the fluorescence signals were detected using an M5e (Molecular Device) instrument (excitation wavelength of 320 nm, emission wavelengths of 620 nm and 665 nm). The inhibition rate for each well was calculated from the fluorescence intensity value of each well: ER (Emission Ratio) = (fluorescence intensity at 665 nm/fluorescence intensity at 620 nm); Inhibition rate = (ERpositive-ERtest compound)/(ERpositive-ERnegative) × 100%, and the $IC_{50}$ values of the compounds were calculated with standard parameter fitting software (GraphPad Prism 8.0) ($IC_{50}$ is the inhibitory concentration at 50% maximum efficacy). The results are shown in Table 1.

Table 1

| Compound No. | ATR/$IC_{50}$/nM |
|---|---|
| **M3** | 7 |
| **M7** | 9 |
| **M15** | 10 |
| **M16** | 445 |
| **M18** | 64 |
| **M1a** | 25 |
| **M1b** | 138 |
| **M2a** | 33 |

(continued)

| Compound No. | ATR/$IC_{50}$/nM |
|---|---|
| **M2b** | 548 |
| **M19** | 15 |
| **M20** | 41 |
| **M21** | 22 |
| **AZD6738** | 15 |

From the data in Table 1, the compounds of the present invention have an excellent effect of inhibiting ATR kinase, which is comparable to or even better than that of the clinically investigational drug **AZD6738**, indicating that the compounds of the present invention can act as an ATR inhibitor selectively on tumor cells and are expected to become excellent potential drugs for tumor treatment.

**Example 10: *In vitro* cell proliferation inhibition assay 1**

22Rv1 cells were purchased from the American Type Culture Collection (ATCC).

The specific steps of the assay are as follows:
22R v 1 cells were cultured in RPMI 1640 medium (containing 10% FBS and 1% penicillin-streptomycin (ps)), and used for the assay when the cell confluence reached more than 85%. About 2000 cells were seeded into each well of a 96-well culture plate and cultured for 24 hours. Different concentrations of the test compounds (0-50 μM) were added to treat the cells. Three parallel replicates were set for each group. Blank (containing the medium only) and control (seeded with cells, but without drug) wells were set up. After culturing for 120 hours, 10 μL of CCK8 solution (Beyotime, #C0037) was added to each well, incubated in the dark for 4 hours, and the OD values were read using a Biotek Synergy H1 multi-function microplate reader.

$$\text{Inhibition rate (\%)} = 100\% \times (\text{control well - test well}) / (\text{control well - blank well})$$

$IC_{50}$ values of the compounds were calculated with standard parameter fitting software (GraphPad Prism 8.0). The results are shown in Table 2.

Table 2

| Compound No. | 22Rv1/$IC_{50}$/μM | Compound No. | 22Rv1/$IC_{50}$/μM |
|---|---|---|---|
| **M3** | 0.21 | **M13** | 1.38 |
| **M4** | 0.65 | **M14** | 1.40 |
| **M5** | 0.44 | **M15** | 0.20 |
| **M6** | 1.33 | **M16** | 4.05 |
| **M7** | 0.22 | **M18** | 0.33 |
| **M8** | 2.40 | **M19** | 0.15 |
| **M9** | 0.97 | **M20** | 0.59 |
| **M10** | 0.37 | **M21** | 4.33 |
| **M11** | 0.50 | **M22** | 4.48 |
| **M12** | 1.71 | | |

The results of the proliferation inhibition assay demonstrate that the compounds of the present invention have a good inhibitory effect on ATM mutated human prostate cancer cell 22Rv1, and some $IC_{50}$ values can be lower than 1 μM, or even lower than 0.3 μM.

**Example 11:** ***In vitro*** **cell proliferation inhibition assay 2**

In this assay, the inhibitory effect of the compounds of the present invention on tumor cell proliferation was studied by detecting the effects of the compounds of the present invention on *in vitro* cell proliferation in tumor cell lines TOV21G and SK-OV-3 (ovarian cancer), Rec-1 (lymphoma), HCT-116 (colon cancer), MDA-MB-231 (breast cancer), NCI-H23 (non-small cell lung cancer), SNU216 (gastric cancer), OS-RC-2 (renal cancer), T24 (bladder cancer), AsPC-1 (pancreatic cancer), M14 (melanoma) and U2OS (osteosarcoma).

TOV21G cells and Rec-1 cells were purchased from the American Type Culture Collection (ATCC), and other cells were from Shanghai Medicilon Biopharmaceutical Co., Ltd.

TOV21G cells were cultured in MCDB105/M199 medium (containing 15% FBS and 1% PS), and were used for the assay when the cell confluence reached more than 85%. About 1000 cells per well were seeded into a 96-well culture plate and cultured for 24 hours. Different concentrations of the test compounds (0-10 μM) were added to treat the cells, and 3 parallel replicate wells were set up for each group. Blank (containing the medium only) and control (seeded with cells, but without drug) wells were set up. After 120 hours of culture, 40 μL of Cell Titer-Glo solution (Promega, #G7573) per well was added, and the cells were incubated for 25 min with shaking in the dark. 100 μL per well was transferred to a 96-well white plate (Corning, #3917), and the luminescence values were read using a Biotek Synergy H1 multi-function microplate reader.

Rec-1 cells were cultured in RPMI1640 medium (containing 10% FBS and 1% ps), and about 6000 cells per well were seeded into a 96-well culture plate. Different concentrations of the test compounds (0-10 μM) were added to treat the cells, and 3 parallel replicate wells were set up for each group. Blank (containing the medium only) and control (seeded with cells but without drug treatment) wells were set up. After 120 h of culture, 40 μL of Cell Titer-Glo solution per well was added, and the cells were incubated for 20 min with shaking in the dark. 100 μL per each well was transferred to a 96-well white plate, and the luminescence values were read using a Biotek Synergy H1 multi-function microplate reader.

The culture and experimental protocols of the other cells referred to the experimental protocols of the above two cell lines, and the conventional culture mediums recommended by ATCC were used as the cell culture mediums.

$$\text{Inhibition rate (\%)} = 100\% \times (\text{control well - test well}) / (\text{control well - blank well})$$

The $IC_{50}$ values of the compounds were calculated with standard parameter fitting software (GraphPad Prism 8.0). The results are shown in Tables 3 and 4.

Table 3

| **Compound No.** | TOV21G /$IC_{50}$/μM | Rec-1 /$IC_{50}$/μM |
|---|---|---|
| **M1a** | 0.68 | 0.89 |
| **M1b** | 2.26 | 1.64 |
| **M2a** | 1.00 | 1.41 |
| **M2b** | 2.72 | 2.16 |

Table 4 Anti-proliferation effects of compound **M3** on various tumor cells.

| Cell line | Tumor type to which the cell belongs | $IC_{50}$/μM |
|---|---|---|
| TOV21G | ovarian cancer | 0.58 |
| Rec-1 | human mantle cell lymphoma | 1.16 |
| SK-OV-3 | ovarian cancer | 1.89 |
| HCT-116 | colon cancer | 1.72 |
| MDA-MB-231 | breast cancer | 1.08 |
| NCI-H23 | non-small cell lung cancer | 1.54 |
| SNU216 | gastric cancer | 1.24 |
| OS-RC-2 | renal cancer | 0.97 |
| T24 | bladder cancer | 0.48 |
| AsPC-1 | pancreatic cancer | 1.57 |

(continued)

| Cell line | Tumor type to which the cell belongs | $IC_{50}$/μM |
|---|---|---|
| M14 | melanoma | 0.88 |
| U2OS | osteosarcoma | 0.69 |

The results of the proliferation inhibition assay demonstrate that the compounds of the present invention have a good inhibitory effect on the human tumor cells studied.

**Example 12: Assay of the stability of the compounds in liver microsomes *in vitro***

The compounds of the present invention were tested for their stability in liver microsomes. The test compounds (final concentration 2.0 nM) was co-incubated with human/mouse liver microsomes in the presence or absence of NADPH, and the concentrations of the compounds in the culture supernatants were measured within 60 minutes. The results for the representative compounds are shown in Table 5 below.

Table 5

| Compound | Human liver microsomes $t_{1/2}$ (min) | Mouse liver microsomes $t_{1/2}$ (min) |
|---|---|---|
| **M3** | 154.32 | 67.77 |
| **M1a** | 12.99 | 37.02 |

The above results show that the molecules of the present invention have very high stability in mouse and human liver microsomes, indicating that their metabolism in the body is relatively slow.

**Example 13: *In vivo* pharmacokinetic experiment of the compounds**

*In vivo* pharmacokinetic studies were conducted for the compounds of the present invention.

Experimental procedures:

Male ICR mice (3 mice/group) were orally gavaged with the test compounds at a dosage of 10 mg/kg. Plasma samples were collected before (0 hour) and after (0.25, 0.5, 1, 2, 4, 6, 8, 24 hours) the administration, and the collected samples were subjected to LC/MS/analysis and data acquisition. The acquisited analytical data were used to calculate relevant pharmacokinetic parameters using Analyst v1.6.2 software (AB Applied Biosystems Company, USA).
The results for the representative compounds are shown in Table 6 below.

Table 6

| Compound No. | Half-life (h) | Maximum Concentration (ng/mL) | Area Under the Curve from 0 to 24 hours (h*ng/mL) |
|---|---|---|---|
| **M3** | 2.0193 | 2924 | 9028 |
| **M1a** | 1.512 | 2386 | 7397 |

The above results indicate that the molecules of the present invention have a very high exposure *in vivo* after oral administration in mice, and are expected to bring excellent anti-tumor effects at a lower dose in clinical.

**Example 14: Study on the selectivity of the compounds for ATR kinase**

The compounds of the present invention were tested for their inhibition on the kinases **(ATM, DNA-PK)** in the same family:
Following the experimental methods reported in the reference documents, the test compounds were diluted in a 3-fold series to 0.51 nM (a total of 10 concentrations) starting from 10 μM, and their inhibitory activity against the kinases ATM[1] and DNA-PK[2] were measured respectively. The results are shown in Table 7 below.

Table 7

| Compound | $IC_{50}$/ATM (nM) | $IC_{50}$/DNAPK (nM) |
|---|---|---|
| M3 | >10000 | 179.6 |
| M1a | >10000 | 60 |

The above results indicate that the compounds of the present invention are highly selective for ATR and have lower inhibitory activity against other kinases in the family, thereby bringing higher safety benefits.

The above content is a further detailed explanation of the present invention in combination with specific preferred embodiments, and it should not be construed to limit the implementation of the present invention to these descriptions. For those skilled in the art to which the present invention pertains, some simple deductions or substitutions can be made without departing from the concept of the present invention, all of which shall be considered within the scope of the present invention.

**References:**

[1] Discovery of Novel 3-Quinoline Carboxamides as Potent, Selective and Orally Bioavailable Inhibitors of Ataxia Telangiectasia Mutated (ATM) Kinase, J. Med. Chem. 2016, 56, 6281-6292;

[2] The Discovery of 7-Methyl-2-[(7-methyl[1,2,4]triazolo[1,5-a]pyridin-6-yl)amino]-9-(tetrahydro-2H-pyran-4-yl)-7,9-dihydro-8H-purin-8-one (AZD7648), a Potent and Selective DNA-Dependent Protein Kinase (DNA-PK) Inhibitor, J. Med. Chem. 2020, 63, 3461-3471.

## Claims

1. A compound represented by general formula (I):

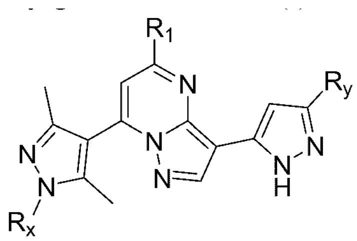

(I)

wherein:

$R_1$ is selected from

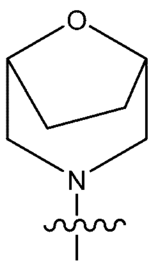, 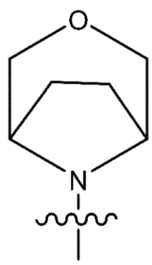, 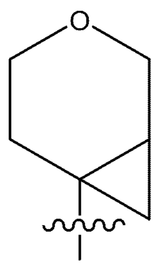, 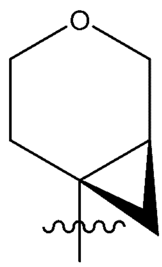, 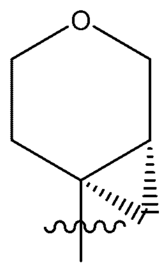,

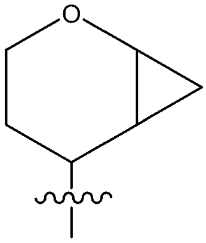 , 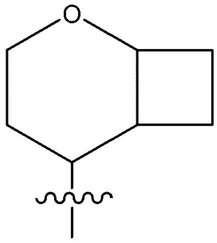 , 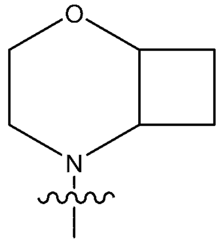 , 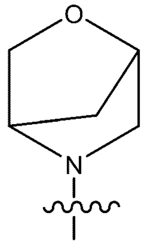 , 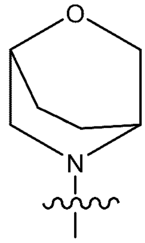 ,

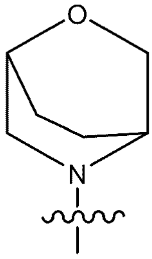 , 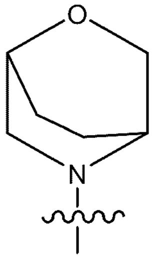 , 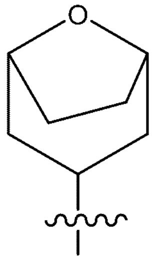 , 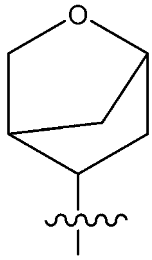 , 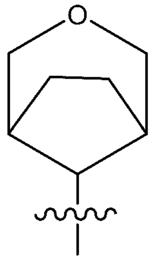 , or 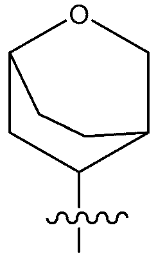 ;

$R_x$ is selected from H, a 5- to 6- membered heteroaryl, or a 5- to 6- membered heterocyclyl, wherein the 5- to 6- membered heteroaryl or the 5- to 6- membered heterocyclyl is optionally substituted with $R_a$;
$R_a$ is selected from H, halogen, -CN, $-NH_2$, $C_{1-4}$ alkyl, $C_{1-4}$ alkoxy, or $C_{1-4}$ haloalkyl; and
$R_y$ is selected from H, halogen, $NH_2$, $C_{1-4}$ alkyl, or $C_{1-4}$ alkoxy, or
a stereoisomer or a pharmaceutically acceptable salt thereof.

**2.** The compound according to claim 1, a stereoisomer or a pharmaceutically acceptable salt thereof, wherein $R_1$ is selected from:

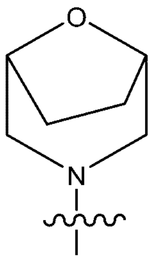 , 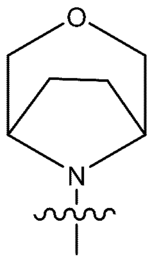 , 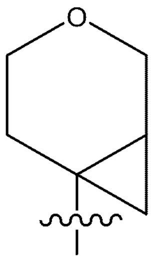 , 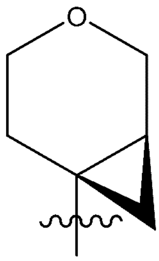 , 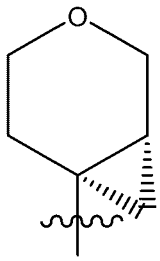 , 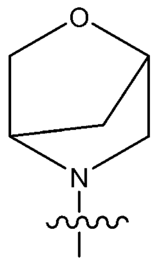 , 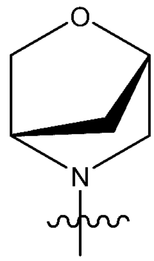 ,

or

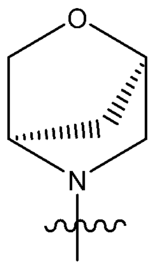

**3.** The compound according to claim 1 or 2, a stereoisomer or a pharmaceutically acceptable salt thereof, wherein:

$R_x$ is selected from H, a 6-membered heteroaryl or a 6-membered heterocyclyl, wherein the 6-membered heteroaryl or the 6-membered heterocyclyl is optionally substituted with $R_a$; and
$R_a$ is selected from H, halogen, -CN, $-NH_2$, $C_{1-4}$ alkyl, $C_{1-4}$ alkoxy, or $C_{1-4}$ haloalkyl.

**4.** The compound according to any one of claims 1 to 3, a stereoisomer or a pharmaceutically acceptable salt thereof, wherein $R_y$ is selected from H or $C_{1-4}$ alkyl.

**5.** The compound according to claim 1, having a structure of general formula (II):

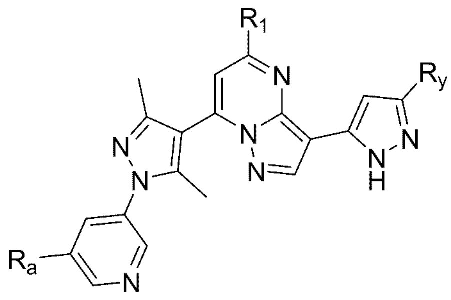

(II)

wherein:

$R_1$ is selected from:

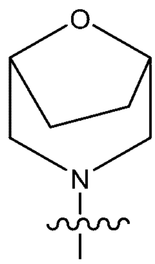
, or
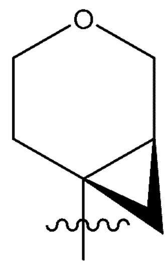
;

$R_a$ is selected from H, halogen, -CN, $-NH_2$, $C_{1-4}$ alkyl, $C_{1-4}$ alkoxy, or $C_{1-4}$ haloalkyl; and
$R_y$ is selected from H or methyl, or
a stereoisomer or a pharmaceutically acceptable salt thereof.

**6.** The compound according to any one of claims 1 to 4, wherein the compound is selected from:

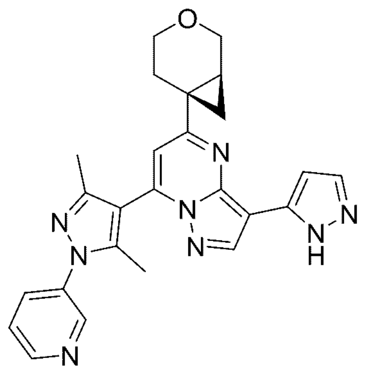
,
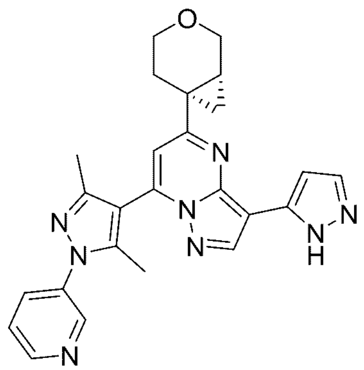
,
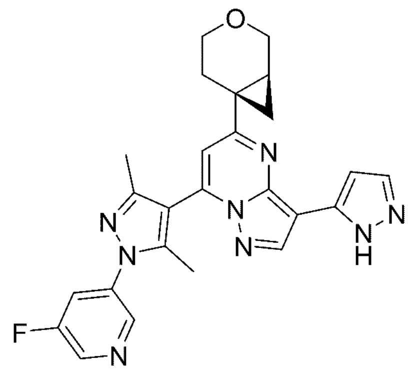
,
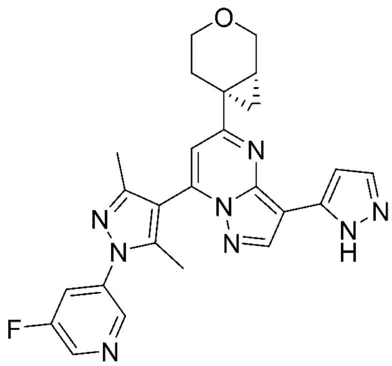
,
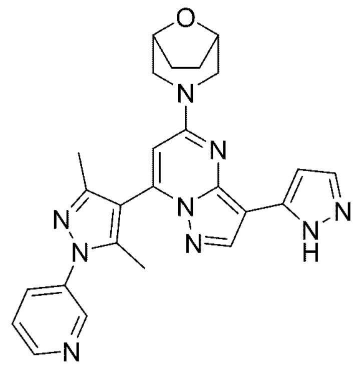
,
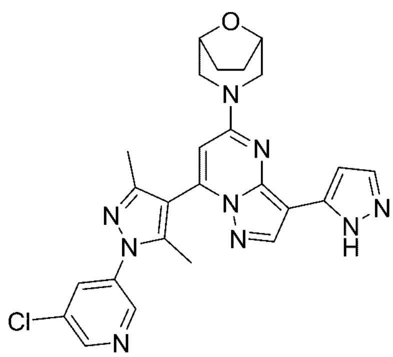
,

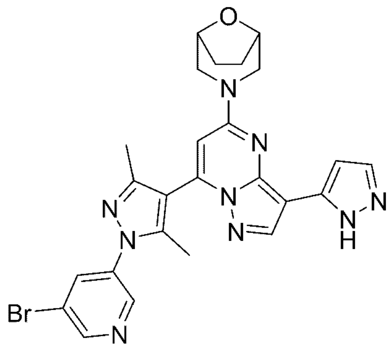,

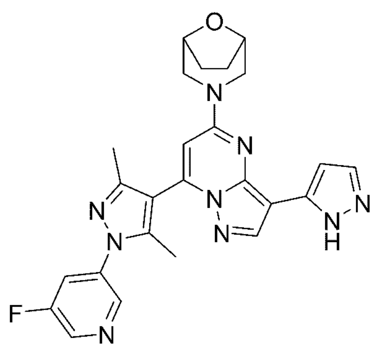,

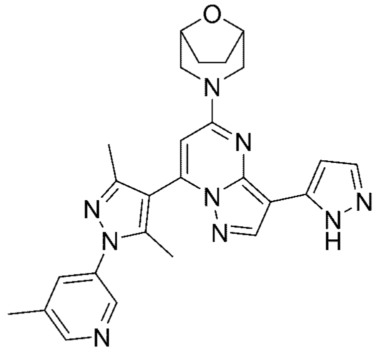,

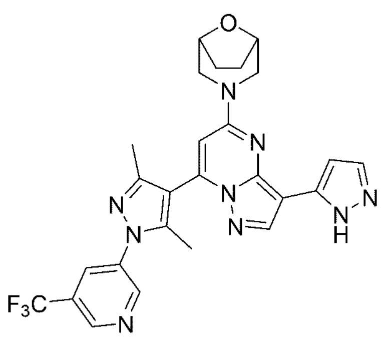,

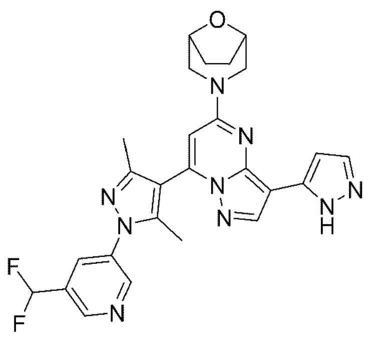,

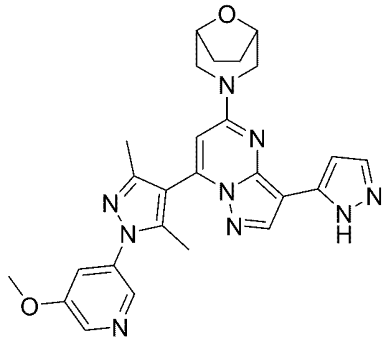,

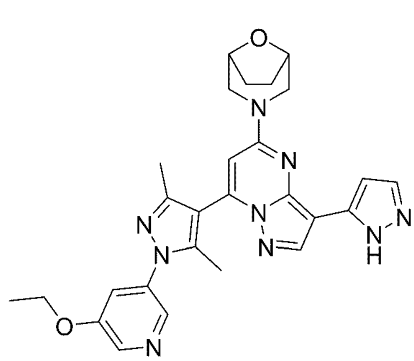,

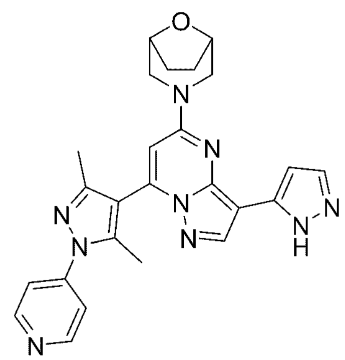,

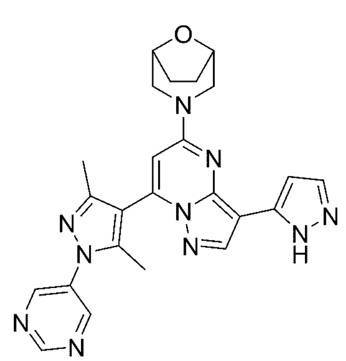,

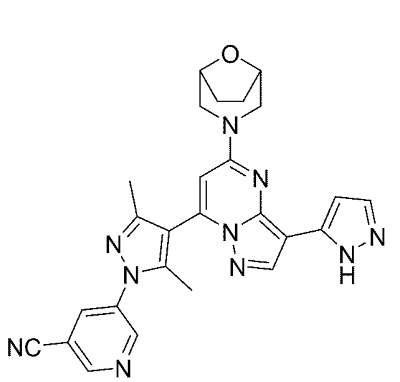,

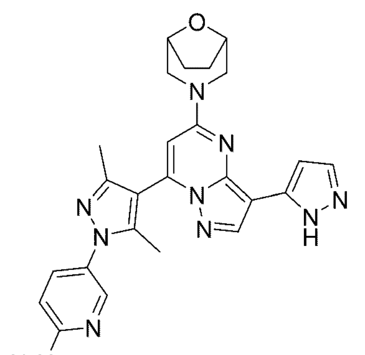,

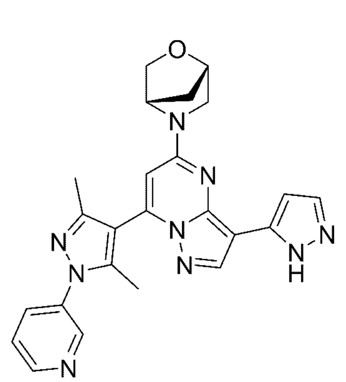,

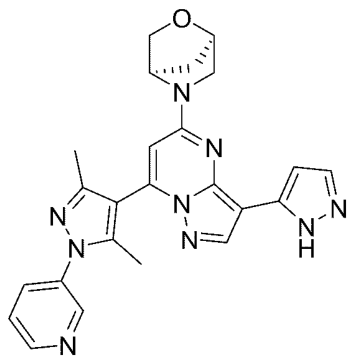
,
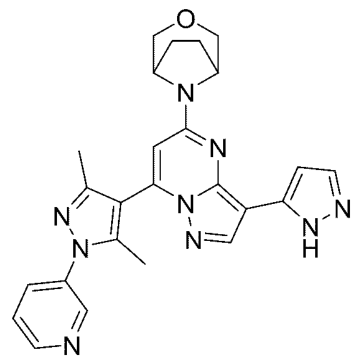
,
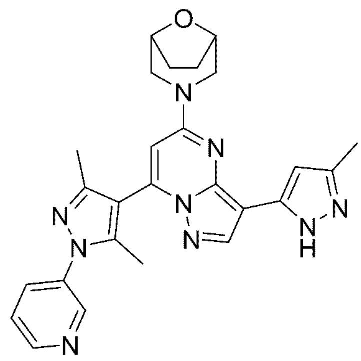
,

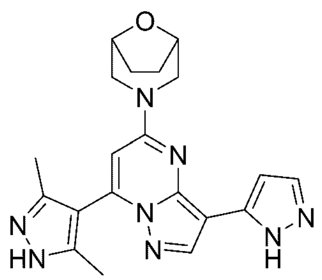
,
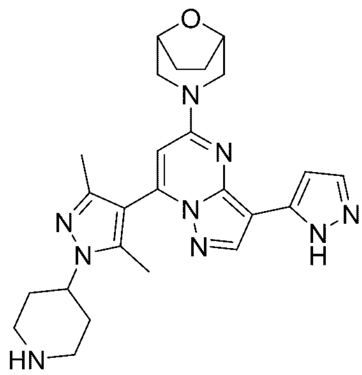
,
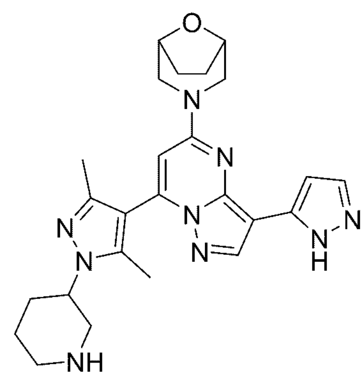
,

or

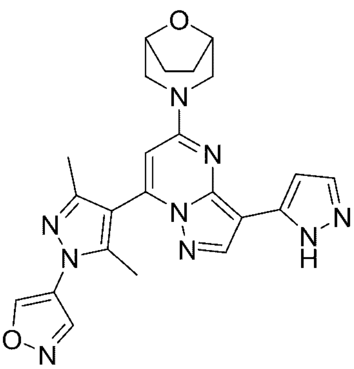
,

or
a stereoisomer or a pharmaceutically acceptable salt thereof.

**7.** A pharmaceutical composition comprising a therapeutically effective amount of the compound according to any one of claims 1 to 6, a stereoisomer or a pharmaceutically acceptable salt thereof, and a pharmaceutically acceptable adjuvant.

**8.** The pharmaceutical composition according to claim 7, further comprising an additional active agent for treating cancer.

**9.** The pharmaceutical composition according to claim 7 or 8, wherein the pharmaceutical composition is formulated as an injection, such as sterile aqueous and non-aqueous solutions, a dispersion, a suspension or an emulsion; a solid oral formulation, such as tablets, capsules, powders, granules or pills; or a liquid oral formulation, such as a solution, an emulsions, a suspension or a syrup.

**10.** Use of the compound according to any one of claims 1 to 6, a stereoisomer or a pharmaceutically acceptable salt

thereof, or the pharmaceutical composition according to any one of claims 7 to 9 in the preparation of a medicament for treating cancer.

**11.** The use according to claim 10, wherein the cancer is selected from the group consisting of breast cancer, kidney cancer, lung cancer, ovarian cancer, bladder cancer, gastric cancer, colorectal cancer, liver cancer, pancreatic cancer, prostate cancer, leukemia, lymphoma, melanoma, myeloma and osteosarcoma.

**INTERNATIONAL SEARCH REPORT**

International application No. **PCT/CN2022/143770**

**A. CLASSIFICATION OF SUBJECT MATTER**

C07D487/04(2006.01)i;C07D498/08(2006.01)i;A61K31/519(2006.01)i;A61K31/5386(2006.01)i;A61P35/00(2006.01)i

According to International Patent Classification (IPC) or to both national classification and IPC

**B. FIELDS SEARCHED**

Minimum documentation searched (classification system followed by classification symbols)

IPC：C07D，A61K31/-，A61P35/-

Documentation searched other than minimum documentation to the extent that such documents are included in the fields searched

Electronic data base consulted during the international search (name of data base and, where practicable, search terms used)

CNKI, CNTXT, CNABS, VEN, 万方数据, WANFANG DATABASE, Caplus, Registry, Marpat: 吡唑并嘧啶, 吡唑并, 嘧啶, ATR, Rad3, 癌, 瘤, pyrazolo, pyrimidin, cancer, tumor, tumour, structure search

**C. DOCUMENTS CONSIDERED TO BE RELEVANT**

| Category* | Citation of document, with indication, where appropriate, of the relevant passages | Relevant to claim No. |
|---|---|---|
| PY | WO 2022028598 A1 (SHANGHAI ANTENGENE CORPORATION LIMITED et al.) 10 February 2022 (2022-02-10)<br>see claims 1-74 | 1-11 |
| PY | WO 2022063308 A1 (MEDSHINE DISCOVERY INC.) 31 March 2022 (2022-03-31)<br>see claims 1-8 | 1-11 |
| Y | CN 113929688 A (BEIJING TIDE PHARMACEUTICAL CO., LTD.) 14 January 2022 (2022-01-14)<br>see entire document, in particular, claims 1-23, and description, embodiments 1-18 | 1-11 |
| Y | CN 108137600 A (UCB BIOPHARMA SPRL et al.) 08 June 2018 (2018-06-08)<br>see claims 1-11, and description, paragraphs 63 and 164 | 1-11 |
| Y | CN 112142744 A (SHANGHAI YINGPAI PHARMACEUTICAL CO., LTD.) 29 December 2020 (2020-12-29)<br>see entire document, in particualar, claims 1-10 | 1-11 |
| Y | WO 2021224636 A1 (ADORX THERAPEUTICS LIMITED) 11 November 2021 (2021-11-11)<br>see claims 1, 18-19, and 22-24, and description, pages 35-37 | 1-11 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 March 2023** | **23 March 2023** |
| Name and mailing address of the ISA/CN<br>**China National Intellectual Property Administration (ISA/CN)**<br>**China No. 6, Xitucheng Road, Jimenqiao, Haidian District, Beijing 100088**<br>Facsimile No. **(86-10)62019451** | Authorized officer<br><br>Telephone No. |

Form PCT/ISA/210 (second sheet) (July 2022)

**INTERNATIONAL SEARCH REPORT**

International application No.
**PCT/CN2022/143770**

**C. DOCUMENTS CONSIDERED TO BE RELEVANT**

| Category* | Citation of document, with indication, where appropriate, of the relevant passages | Relevant to claim No. |
|---|---|---|
| Y | WO 2021127190 A1 (KYMERA THERAPEUTICS, INC.) 24 June 2021 (2021-06-24) see claims 1-17, and description, paragraphs 0020 and 0099 | 1-11 |

Form PCT/ISA/210 (second sheet) (July 2022)

**INTERNATIONAL SEARCH REPORT**
**Information on patent family members**

International application No.
**PCT/CN2022/143770**

| Patent document cited in search report | Publication date (day/month/year) | Patent family member(s) | Publication date (day/month/year) |
|---|---|---|---|
| WO 2022028598 A1 | 10 February 2022 | TW 202220993 A | 01 June 2022 |
| WO 2022063308 A1 | 31 March 2022 | None | |
| CN 113929688 A | 14 January 2022 | WO 2022012484 A1 | 20 January 2022 |
| | | TW 202202506 A | 16 January 2022 |
| | | HK 40064066 A0 | 30 June 2023 |
| CN 108137600 A | 08 June 2018 | GB 201517263 D0 | 11 November 2015 |
| | | BR 112018006135 A2 | 23 October 2018 |
| | | EP 3356367 A1 | 08 August 2018 |
| | | CA 2998802 A1 | 06 April 2017 |
| | | JP 2018529725 A | 11 October 2018 |
| | | WO 2017055305 A1 | 06 April 2017 |
| | | EA 201890827 A1 | 31 October 2018 |
| | | US 2018298009 A1 | 18 October 2018 |
| | | IN 201817009195 A | 15 June 2018 |
| CN 112142744 A | 29 December 2020 | CA 3144806 A1 | 30 December 2020 |
| | | WO 2020259601 A1 | 30 December 2020 |
| | | BR 112021026498 A2 | 08 February 2022 |
| | | AU 2020306082 A1 | 17 February 2022 |
| | | SG 11202113683 A | 25 February 2022 |
| | | KR 20220035917 A | 22 March 2022 |
| | | CN 114423756 A | 29 April 2022 |
| | | EP 3997087 A1 | 18 May 2022 |
| | | US 2022267332 A1 | 25 August 2022 |
| | | JP 2022541127 W | 25 August 2022 |
| WO 2021224636 A1 | 11 November 2021 | IL 297963 A | 01 January 2023 |
| | | CO 2022017618 A2 | 16 February 2023 |
| | | CA 3181354 A1 | 11 November 2021 |
| | | BR 112022022437 A2 | 03 January 2023 |
| | | AU 2021266433 A1 | 15 December 2022 |
| | | GB 202217519 D0 | 04 January 2023 |
| | | GB 2609879 A | 15 February 2023 |
| WO 2021127190 A1 | 24 June 2021 | BR 112022011651 A2 | 23 August 2022 |
| | | AU 2020407200 A1 | 21 July 2022 |
| | | EP 4076520 A1 | 26 October 2022 |
| | | CO 2022008406 A2 | 08 July 2022 |
| | | TW 202136251 A | 01 October 2021 |
| | | CA 3161878 A1 | 24 June 2021 |
| | | IL 293917 A | 01 August 2022 |
| | | KR 20220145325 A | 28 October 2022 |
| | | US 2021228562 A1 | 29 July 2021 |
| | | CN 115052627 A | 13 September 2022 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Discovery of Novel 3-Quinoline Carboxamides as Potent, Selective and Orally Bioavailable Inhibitors of Ataxia Telangiectasia Mutated (ATM) Kinase. *J. Med. Chem.*, 2016, vol. 56, 6281-6292
- The Discovery of 7-Methyl-2-[(7-methyl[1,2,4]triazolo[1,5-a]pyridin-6-yl)amino]-9-(tetrahydro-2H-pyran-4-yl)-7,9-dihydro-8H-purin-8-one (AZD7648), a Potent and Selective DNA-Dependent Protein Kinase (DNA-PK) Inhibitor. *J. Med. Chem.*, 2020, vol. 63, 3461-3471